# EUROPEAN PATENT APPLICATION

(11) **EP 2 565 264 A1**
(43) Date of publication of application: **06.03.2013**
(21) Application number: 11306080.0
(22) Date of filing: 29.08.2011
(51) Int. Cl.: C12N 5/0775

(54) **Method for preparing induced paraxial mesoderm progenitor (iPAM) cells and their use**

(71) Applicant: INSERM (Institut National de la Santé et de la Recherche Médicale), 75013 Paris (FR); Centre National de la Recherche Scientifique - CNRS, 75016 Paris Cedex 16 (FR); Université de Strasbourg, 67000 Strasbourg (FR); Association Française contre les Myopathies, 75651 Paris Cedex 13 (FR)
(72) Inventor: Pourquie, Olivier, 67404 Illkirch Cedex (FR); Chal, Jérôme, 67404 Illkirch Cedex (FR)
(74) Representative: Hirsch, Denise Marie

(57) **Abstract**

The present invention relates to an *ex vivo* method for preparing a population of induced paraxial mesoderm progenitor (iPAM) cells, said method comprising the step of culturing pluripotent cells in an appropriate culture medium comprising an effective amount of an activator of the Wnt signalling pathway.

SEQUENCE LISTING

<110>  INSERM

<120>  METHOD FOR PREPARING INDUCED PRESOMITIC MESODERM (IPM) CELLS AND
       THEIR USE

<130>  BIO11079 POURQUIE

<160>  9

<170>  PatentIn version 3.3

<210>  1
<211>  272
<212>  PRT
<213>  Homo sapiens

<400>  1

Met His Leu Arg Leu Ile Ser Trp Leu Phe Ile Ile Leu Asn Phe Met
1               5                   10                  15

Glu Tyr Ile Gly Ser Gln Asn Ala Ser Arg Gly Arg Arg Gln Arg Arg
            20                  25                  30

Met His Pro Asn Val Ser Gln Gly Cys Gln Gly Gly Cys Ala Thr Cys
        35                  40                  45

Ser Asp Tyr Asn Gly Cys Leu Ser Cys Lys Pro Arg Leu Phe Phe Ala
    50                  55                  60

Leu Glu Arg Ile Gly Met Lys Gln Ile Gly Val Cys Leu Ser Ser Cys
65                  70                  75                  80

Pro Ser Gly Tyr Tyr Gly Thr Arg Tyr Pro Asp Ile Asn Lys Cys Thr
                85                  90                  95

Lys Cys Lys Ala Asp Cys Asp Thr Cys Phe Asn Lys Asn Phe Cys Thr
            100                 105                 110

Lys Cys Lys Ser Gly Phe Tyr Leu His Leu Gly Lys Cys Leu Asp Asn
        115                 120                 125

Cys Pro Glu Gly Leu Glu Ala Asn Asn His Thr Met Glu Cys Val Ser
    130                 135                 140

Ile Val His Cys Glu Val Ser Glu Trp Asn Pro Trp Ser Pro Cys Thr
145                 150                 155                 160

Lys Lys Gly Lys Thr Cys Gly Phe Lys Arg Gly Thr Glu Thr Arg Val
                165                 170                 175

Arg Glu Ile Ile Gln His Pro Ser Ala Lys Gly Asn Leu Cys Pro Pro

180                 185                 190

Thr Asn Glu Thr Arg Lys Cys Thr Val Gln Arg Lys Lys Cys Gln Lys
        195                 200                 205

Gly Glu Arg Gly Lys Lys Gly Arg Glu Arg Lys Arg Lys Lys Pro Asn
    210                 215                 220

Lys Gly Glu Ser Lys Glu Ala Ile Pro Asp Ser Lys Ser Leu Glu Ser
225                 230                 235                 240

Ser Lys Glu Ile Pro Glu Gln Arg Glu Asn Lys Gln Gln Gln Lys Lys
                245                 250                 255

Arg Lys Val Gln Asp Lys Gln Lys Ser Val Ser Val Ser Thr Val His
            260                 265                 270

<210>  2
<211>  277
<212>  PRT
<213>  Mus musculus

<400>  2

Met His Leu Arg Leu Ile Ser Cys Phe Phe Ile Ile Leu Asn Phe Met
1               5                   10                  15

Glu Tyr Ile Gly Ser Gln Asn Ala Ser Arg Gly Arg Arg Gln Arg Arg
            20                  25                  30

Met His Pro Asn Val Ser Gln Gly Cys Gln Gly Gly Cys Ala Thr Cys
        35                  40                  45

Ser Asp Tyr Asn Gly Cys Leu Ser Cys Lys Pro Arg Leu Phe Phe Val
    50                  55                  60

Leu Glu Arg Ile Gly Met Lys Gln Ile Gly Val Cys Leu Ser Ser Cys
65                  70                  75                  80

Pro Ser Gly Tyr Tyr Gly Thr Arg Tyr Pro Asp Ile Asn Lys Cys Thr
                85                  90                  95

Lys Cys Lys Val Asp Cys Asp Thr Cys Phe Asn Lys Asn Phe Cys Thr
            100                 105                 110

Lys Cys Lys Ser Gly Phe Tyr Leu His Leu Gly Lys Cys Leu Asp Ser
        115                 120                 125

Cys Pro Glu Gly Leu Glu Ala Asn Asn His Thr Met Glu Cys Val Ser
    130                 135                 140

Ile Val His Cys Glu Ala Ser Glu Trp Ser Pro Trp Ser Pro Cys Met
145                 150                 155                 160

Lys Lys Gly Lys Thr Cys Gly Phe Lys Arg Gly Thr Glu Thr Arg Val
                165                 170                 175

Arg Asp Ile Leu Gln His Pro Ser Ala Lys Gly Asn Leu Cys Pro Pro
            180                 185                 190

Thr Ser Glu Thr Arg Thr Cys Ile Val Gln Arg Lys Lys Cys Ser Lys
        195                 200                 205

Gly Glu Arg Gly Lys Lys Gly Arg Glu Arg Lys Arg Lys Lys Leu Asn
    210                 215                 220

Lys Glu Glu Arg Lys Glu Thr Ser Ser Ser Ser Asp Ser Lys Gly Leu
225                 230                 235                 240

Glu Ser Ser Ile Glu Thr Pro Asp Gln Gln Glu Asn Lys Glu Arg Gln
                245                 250                 255

Gln Gln Gln Lys Arg Arg Ala Arg Asp Lys Gln Gln Lys Ser Val Ser
            260                 265                 270

Val Ser Thr Val His
        275

<210>  3
<211>  243
<212>  PRT
<213>  Homo sapiens

<400>  3

Met Gln Phe Arg Leu Phe Ser Phe Ala Leu Ile Ile Leu Asn Cys Met
1               5                   10                  15

Asp Tyr Ser His Cys Gln Gly Asn Arg Trp Arg Arg Ser Lys Arg Ala
            20                  25                  30

Ser Tyr Val Ser Asn Pro Ile Cys Lys Gly Cys Leu Ser Cys Ser Lys
        35                  40                  45

Asp Asn Gly Cys Ser Arg Cys Gln Gln Lys Leu Phe Phe Phe Leu Arg
    50                  55                  60

Arg Glu Gly Met Arg Gln Tyr Gly Glu Cys Leu His Ser Cys Pro Ser
65                  70                  75                  80

Gly Tyr Tyr Gly His Arg Ala Pro Asp Met Asn Arg Cys Ala Arg Cys

85                  90                  95

Arg Ile Glu Asn Cys Asp Ser Cys Phe Ser Lys Asp Phe Cys Thr Lys
            100                 105                 110

Cys Lys Val Gly Phe Tyr Leu His Arg Gly Arg Cys Phe Asp Glu Cys
        115                 120                 125

Pro Asp Gly Phe Ala Pro Leu Glu Glu Thr Met Glu Cys Val Glu Gly
    130                 135                 140

Cys Glu Val Gly His Trp Ser Glu Trp Gly Thr Cys Ser Arg Asn Asn
145                 150                 155                 160

Arg Thr Cys Gly Phe Lys Trp Gly Leu Glu Thr Arg Thr Arg Gln Ile
                165                 170                 175

Val Lys Lys Pro Val Lys Asp Thr Ile Leu Cys Pro Thr Ile Ala Glu
            180                 185                 190

Ser Arg Arg Cys Lys Met Thr Met Arg His Cys Pro Gly Gly Lys Arg
        195                 200                 205

Thr Pro Lys Ala Lys Glu Lys Arg Asn Lys Lys Lys Lys Arg Lys Leu
    210                 215                 220

Ile Glu Arg Ala Gln Glu Gln His Ser Val Phe Leu Ala Thr Asp Arg
225                 230                 235                 240

Ala Asn Gln

<210>  4
<211>  243
<212>  PRT
<213>  Mus musculus

<400>  4

Met Arg Phe Cys Leu Phe Ser Phe Ala Leu Ile Ile Leu Asn Cys Met
1               5                   10                  15

Asp Tyr Ser Gln Cys Gln Gly Asn Arg Trp Arg Arg Asn Lys Arg Ala
            20                  25                  30

Ser Tyr Val Ser Asn Pro Ile Cys Lys Gly Cys Leu Ser Cys Ser Lys
        35                  40                  45

Asp Asn Gly Cys Ser Arg Cys Gln Gln Lys Leu Phe Phe Phe Leu Arg
    50                  55                  60

Arg Glu Gly Met Arg Gln Tyr Gly Glu Cys Leu His Ser Cys Pro Ser
65                  70                  75                  80

Gly Tyr Tyr Gly His Arg Ala Pro Asp Met Asn Arg Cys Ala Arg Cys
                85                  90                  95

Arg Ile Glu Asn Cys Asp Ser Cys Phe Ser Lys Asp Phe Cys Thr Lys
            100                 105                 110

Cys Lys Val Gly Phe Tyr Leu His Arg Gly Arg Cys Phe Asp Glu Cys
        115                 120                 125

Pro Asp Gly Phe Ala Pro Leu Asp Glu Thr Met Glu Cys Val Glu Gly
    130                 135                 140

Cys Glu Val Gly His Trp Ser Glu Trp Gly Thr Cys Ser Arg Asn Asn
145                 150                 155                 160

Arg Thr Cys Gly Phe Lys Trp Gly Leu Glu Thr Arg Thr Arg Gln Ile
                165                 170                 175

Val Lys Lys Pro Ala Lys Asp Thr Ile Pro Cys Pro Thr Ile Ala Glu
            180                 185                 190

Ser Arg Arg Cys Lys Met Ala Met Arg His Cys Pro Gly Gly Lys Arg
        195                 200                 205

Thr Pro Lys Ala Lys Glu Lys Arg Asn Lys Lys Lys Arg Arg Lys Leu
    210                 215                 220

Ile Glu Arg Ala Gln Glu Gln His Ser Val Phe Leu Ala Thr Asp Arg
225                 230                 235                 240

Val Asn Gln

<210>  5
<211>  292
<212>  PRT
<213>  Homo sapiens

<400>  5

Met His Leu Arg Leu Ile Ser Trp Leu Phe Ile Ile Leu Asn Phe Met
1               5                   10                  15

Glu Tyr Ile Gly Ser Gln Asn Ala Ser Arg Gly Arg Arg Gln Arg Arg
            20                  25                  30

Met His Pro Asn Val Ser Gln Gly Cys Gln Gly Gly Cys Ala Thr Cys

        35                  40                  45

Ser Asp Tyr Asn Gly Cys Leu Ser Cys Lys Pro Arg Leu Phe Phe Ala
    50                  55                  60

Leu Glu Arg Ile Gly Met Lys Gln Ile Gly Val Cys Leu Ser Ser Cys
65                  70                  75                  80

Pro Ser Gly Tyr Tyr Gly Thr Arg Tyr Pro Asp Ile Asn Lys Cys Thr
                85                  90                  95

Lys Cys Lys Ala Asp Cys Asp Thr Cys Phe Asn Lys Asn Phe Cys Thr
            100                 105                 110

Lys Cys Lys Ser Gly Phe Tyr Leu His Leu Gly Lys Cys Leu Asp Asn
        115                 120                 125

Cys Pro Glu Gly Leu Glu Ala Asn Asn His Thr Met Glu Cys Val Ser
    130                 135                 140

Ile Val His Cys Glu Val Ser Glu Trp Asn Pro Trp Ser Pro Cys Thr
145                 150                 155                 160

Lys Lys Gly Lys Thr Cys Gly Phe Lys Arg Gly Thr Glu Thr Arg Val
                165                 170                 175

Arg Glu Ile Ile Gln His Pro Ser Ala Lys Gly Asn Leu Cys Pro Pro
            180                 185                 190

Thr Asn Glu Thr Arg Lys Cys Thr Val Gln Arg Lys Lys Cys Gln Lys
        195                 200                 205

Gly Glu Arg Gly Lys Lys Gly Arg Glu Arg Lys Arg Lys Lys Pro Asn
    210                 215                 220

Lys Gly Glu Ser Lys Glu Ala Ile Pro Asp Ser Lys Ser Leu Glu Ser
225                 230                 235                 240

Ser Lys Glu Ile Pro Glu Gln Arg Glu Asn Lys Gln Gln Gln Lys Lys
                245                 250                 255

Arg Lys Val Gln Asp Lys Gln Lys Ser Gly Ile Glu Val Thr Leu Ala
            260                 265                 270

Glu Gly Leu Thr Ser Val Ser Gln Arg Thr Gln Pro Thr Pro Cys Arg
        275                 280                 285

Arg Arg Tyr Leu
    290

<210>  6
<211>  176
<212>  PRT
<213>  Homo sapiens

<400>  6

Met Arg Gln Tyr Gly Glu Cys Leu His Ser Cys Pro Ser Gly Tyr Tyr
1               5                   10                  15

Gly His Arg Ala Pro Asp Met Asn Arg Cys Ala Arg Cys Arg Ile Glu
            20                  25                  30

Asn Cys Asp Ser Cys Phe Ser Lys Asp Phe Cys Thr Lys Cys Lys Val
        35                  40                  45

Gly Phe Tyr Leu His Arg Gly Arg Cys Phe Asp Glu Cys Pro Asp Gly
    50                  55                  60

Phe Ala Pro Leu Glu Glu Thr Met Glu Cys Val Glu Gly Cys Glu Val
65                  70                  75                  80

Gly His Trp Ser Glu Trp Gly Thr Cys Ser Arg Asn Asn Arg Thr Cys
                85                  90                  95

Gly Phe Lys Trp Gly Leu Glu Thr Arg Thr Arg Gln Ile Val Lys Lys
            100                 105                 110

Pro Val Lys Asp Thr Ile Leu Cys Pro Thr Ile Ala Glu Ser Arg Arg
        115                 120                 125

Cys Lys Met Thr Met Arg His Cys Pro Gly Gly Lys Arg Thr Pro Lys
    130                 135                 140

Ala Lys Glu Lys Arg Asn Lys Lys Lys Lys Arg Lys Leu Ile Glu Arg
145                 150                 155                 160

Ala Gln Glu Gln His Ser Val Phe Leu Ala Thr Asp Arg Ala Asn Gln
                165                 170                 175


<210>  7
<211>  177
<212>  PRT
<213>  Homo sapiens

<400>  7

Phe Arg Leu Phe Ser Phe Ala Leu Ile Ile Leu Asn Cys Met Asp Tyr
1               5                   10                  15

Ser His Cys Gln Gly Asn Arg Trp Arg Arg Ser Lys Arg Gly Cys Arg

20                  25                  30

Ile Glu Asn Cys Asp Ser Cys Phe Ser Lys Asp Phe Cys Thr Lys Cys
        35                  40                  45

Lys Val Gly Phe Tyr Leu His Arg Gly Arg Cys Phe Asp Glu Cys Pro
    50                  55                  60

Asp Gly Phe Ala Pro Leu Glu Glu Thr Met Glu Cys Val Gly Cys Glu
65                  70                  75                  80

Val Gly His Trp Ser Glu Trp Gly Thr Cys Ser Arg Asn Asn Arg Thr
                85                  90                  95

Cys Gly Phe Lys Trp Gly Leu Glu Thr Arg Thr Arg Gln Ile Val Lys
            100                 105                 110

Lys Pro Val Lys Asp Thr Ile Leu Cys Pro Thr Ile Ala Glu Ser Arg
        115                 120                 125

Arg Cys Lys Met Thr Met Arg His Cys Pro Gly Gly Lys Arg Thr Pro
    130                 135                 140

Lys Ala Lys Glu Lys Arg Asn Lys Lys Lys Lys Arg Lys Leu Ile Glu
145                 150                 155                 160

Arg Ala Gln Glu Gln His Ser Val Phe Leu Ala Thr Asp Arg Ala Asn
                165                 170                 175

Gln

<210>  8
<211>  567
<212>  DNA
<213>  Mus musculus

<400>  8
atggacaacc tgggtgagac cttcctcagc ctggaggatg gcctggactc ttctgacacc      60

gctggtctgc tggcctcctg ggactggaaa agcagagcca ggcccttgga gctggtccag     120

gagtccccca ctcaaagcct ctccccagct ccttctctgg agtcctactc tgaggtcgca     180

ctgccctgcg ggcacagtgg ggccagcaca ggaggcagcg atggctacgg cagtcacgag     240

gctgccggct tagtcgagct ggattacagc atgttggctt ttcaacctcc ctatctacac     300

actgctggtg gcctcaaagg ccagaaaggc agcaaagtca agatgtctgt ccagcggaga     360

cggaaggcca gcgagagaga gaaactcagg atgcggacct tagccgatgc cctccacacg     420

ctccggaatt acctgccgcc tgtctacagc cagagaggcc aaccgctcac caagatccag     480

acactcaagt acaccatcaa gtacatcggg gaactcacag acctcctcaa cagcagcggg 540

agagagccca ggccacagag tgtgtga 567

<210> 9
<211> 582
<212> DNA
<213> Homo sapiens

<400> 9
atggacaacc tgcgcgagac tttcctcagc ctcgaggatg gcttgggctc ctctgacagc 60

cctggcctgc tgtcttcctg ggactggaag gacagggcag ggccctttga gctgaatcag 120

gcctccccct ctcagagcct ttccccggct ccatcgctgg aatcctattc ttcttctccc 180

tgtccagctg tggctgggct gccctgtgag cacggcgggg ccagcagtgg gggcagcgaa 240

ggctgcagtg tcggtggggc cagtggcctg gtagaggtgg actacaatat gttagctttc 300

cagcccaccc accttcaggg cggtggtggc cccaaggccc agaagggcac caaagtcagg 360

atgtctgtcc agcggaggcg gaaagccagc gagagggaga agctcaggat gaggaccttg 420

gcagatgccc tgcacaccct ccggaattac ctgccacctg tctacagcca gagaggccag 480

cctctcacca agatccagac actcaagtac accatcaagt acatcgggga actcacagac 540

ctccttaacc gcggcagaga gcccagagcc cagagcgcgt ga 582

## Description

### FIELD OF THE INVENTION:

The present invention relates to an *ex vivo* method for preparing a population of induced paraxial mesoderm progenitor (iPAM) cells, said method comprising the step of culturing pluripotent cells in an appropriate culture medium comprising an effective amount of an activator of the Wnt signalling pathway.

### BACKGROUND OF THE INVENTION:

Embryonic stem (ES) cell research offers unprecedented potential for understanding fundamental developmental processes, such as lineage differentiation. Embryonic stem cell lines are derived from early embryos and are characterized by their ability to self-renew, that is, to be maintained indefinitely in a proliferative and undifferentiated state in culture. ES cells are also pluripotent, meaning they retain the capacity to differentiate into the three embryonic lineages: ectoderm, mesoderm and endoderm plus all of their derivatives (Chambers, 2004). The recent development of reprogramming technologies now allows ES-like stem cells to be generated from somatic cells, such as fibroblasts. Introduction into somatic cells of a small set of specific transcription factors - Oct4, Sox2, c-Myc, and Klf4 in the mouse (Takahashi and Yamanaka, 2006) and human (Park et al., 2008b; Takahashi et al., 2007), or Oct4, Sox2, Nanog and Lin28 in human (Yu et al., 2007) - can reprogram various differentiated cell types to an ES-like stem cell state (induced pluripotent stem cells or iPS). This strategy now allows the generation of ES-like cell lines from individual patients and, thus, offers the possibility to create highly relevant *in vitro* models of human genetic diseases. Such reprogrammed cell lines have already been generated from patients with a variety of diseases, such as Duchenne Muscular Dystrophy or Amyotrophic lateral sclerosis (ALS) and differentiation of the reprogrammed cells into the deficient tissue has been achieved for iPS cells from patients affected with several diseases such as ALS, thus, demonstrating the feasibility of the approach (Dimos et al., 2008; Park et al., 2008a).

Whereas some lineages such as cardiac myocytes or neurons are easily generated *in vitro* from ES cells, differentiating paraxial mesoderm derivatives such as skeletal muscle, dermis, cartilage or bone from ES or iPS cells has proven to be challenging. Given the promises offered by cellular replacement therapy for the cure of some muscular degenerative diseases or for orthopaedic surgery, the development of protocols for production of precursors of muscle and skeletal lineages is of key importance. In the embryo, the muscles, the dorsal dermis and the axial skeleton of the body derive from the paraxial mesoderm and more specifically from multipotent precursors forming the presomitic mesoderm (PSM). These precursors are characterized by expression of the genes Brachyury (T), Tbx6 and Mesogenin 1 (Msgn1) (Chapman et al., 1996; Yoon and Wold, 2000) and they mostly differentiate into skeletal muscles, dermis, skeletal lineages, as well as in a variety of other derivatives including adipocytes and endothelial cells. In the mouse embryo, Rspo3 (also called Cristin1, Thsd2) is strongly expressed in the PSM and somites, as well as later in condensing mesenchymal cells, (Kazanskaya et al., 2004; Nam et al., 2007). R-spondins (Rspo1 to 4 genes) are secreted molecules containing a thrombospondin domain that can activate canonical Wnt signaling and Beta-Catenin, via the Fzd/LRP/Lgr4/Lgr5 co-receptor complex (Carmon et al., 2011; de Lau et al., 2011; Kim et al., 2008; Nam et al., 2006), but they were also shown to bind Syndecan4 and induce Wnt/PCP signalling (Ohkawara et al., 2011). Interestingly, biochemical assays show that Rspo2 and 3 are more potent to activate Wnt signaling than Rspo1 and 4 (Kim et al., 2008). R-spondins have also been shown to be implicated in bone formation and chondrogenesis (Hankenson et al., 2010; Jin et al., 2011; Ohkawara et al., 2011), myogenesis (Han et al., 2011; Kazanskaya et al., 2004) and angiogenesis (Kazanskaya et al., 2008).

Differentiation of ES cells into paraxial mesoderm and its derivatives is highly inefficient *in vitro.* Limited spontaneous skeletal muscle differentiation has been described following culture of mouse embryoid bodies and DMSO treatment (Dinsmore et al., 1996; Rohwedel et al., 1994), or Retinoic acid treatment (Kennedy et al., 2009). Two distinct strategies to differentiate mouse and human ES cells *in vitro* to the muscle lineage have been reported. The first one involves the sorting of precursors using surface markers. For instance, Studer's group reported the isolation of human ES cells-derived CD73+ mesenchymal precursors and their subsequent differentiation into skeletal muscle following a culture period in serum containing medium (Barberi et al., 2007). The antibody against satellite cells SM/C-2.6 was also used to isolate myogenic cells differentiated from mouse ES and iPS cells (Fukada et al., 2004; Mizuno et al., 2010). Finally, mesoderm precursors differentiated from mouse ES cells were also isolated based on their expression of other surface markers such as the Platelet derived growth factor receptor alpha (PDGFRa) or Vascular endothelial growth factor receptor 2 (VEGFR2) (Sakurai et al., 2009; Sakurai et al., 2008). The second strategy is based on forced expression of the transcription factors Pax3 or MyoD, or of the secreted factor Insulin Growth Factor 2 (IGF-2) in mouse ES cells (Darabi et al., 2008; Darabi et al., 2011; Dekel et al., 1992; Prelle et al., 2000; Shani et al., 1992). However, these strategies show either limited efficiency or require introduction of exogenous DNA in the ES cells and the differentiated cells often show limited proliferation and engraftment potential.

Therefore, there is a need to develop better ES and iPS cell differentiation strategies to produce muscle cells and paraxial mesoderm derived lineages for the development of applications in regenerative medicine.

The present invention fulfils this need by providing a method for preparing multipotent progenitor cell lines expressing markers of the paraxial mesoderm progenitors and referred to as induced Paraxial Mesoderm progenitor cells or iPAM to distinguish them from the natural embryo Paraxial Mesoderm progenitor cells. Like their *in vivo* counterpart, the iPAM cells are capable of giving rise to cell lineages of the muscular, skeletal (bone and cartilage) or dermal tissue, and derivatives such as adipocytes and endothelium. The inventors have shown that embryonic stem cells or pluripotent reprogrammed cells (iPS) can be differentiated into iPAM cells using a limited number of factors. In particular, the inventors have made the surprising finding that it is possible to efficiently obtain iPAM cells by treatment with only one factor, without any genetic modification of the target cells. They have shown that the obtained iPAM cells exhibit characteristics of endogenous Paraxial mesoderm progenitor cells. To the applicant's knowledge, the invention is the first description of a method for obtaining unlimited amounts of cells suitable for use as progenitor cells for regenerating either muscle, skeletal, adipose or dermal tissues and paraxial mesoderm derived endothelium. Therefore the invention is highly useful in particular in regenerative medicine.

### SUMMARY OF THE INVENTION:

Thus, the present invention relates to an *ex vivo* method for preparing a population of induced paraxial mesoderm progenitor (iPAM) cells, said method comprising the step of culturing pluripotent cells in an appropriate culture medium comprising an effective amount of an activator of the Wnt signalling pathway.

More particularly, the invention relates to an *ex vivo* method for preparing a population of induced paraxial mesoderm progenitor (iPAM) cells, said method comprising the step of culturing pluripotent cells in an appropriate culture medium comprising an effective amount of a member of the R-spondin family.

### DETAILED DESCRIPTION OF THE INVENTION:

### Method for preparing iPAM cells

A first aspect of the invention relates to an *ex vivo* method for preparing a population of induced Paraxial Mesoderm progenitor (iPAM) cells, said method comprising the step of culturing pluripotent cells in an appropriate culture medium comprising an effective amount of an activator of the Wnt signalling pathway.

As used herein, the term "Wnt signalling pathway" denotes a signalling pathway which may be divided in two pathways: the "canonical Wnt/beta catenin signalling pathway" and the "Wnt/PCP signalling pathway". As used herein, the term "canonical Wnt/beta catenin signalling pathway" or "Wnt/PCP signalling pathway" in its general meaning denotes a network of proteins and other bioactive molecules (lipids, ions, sugars...) best known for their roles in embryogenesis and cancer, but also involved in normal physiological processes in adult animals. The "canonical Wnt/beta catenin signalling pathway" is characterized by a Wnt dependant inhibition of glycogen synthase kinase 3ß (GSK-3ß), leading to a subsequent stabilization of ß catenin, which then translocates to the nucleus to act as a transcription factor. The "Wnt/PCP signalling pathway" does not involve GSK-3ß or ß-catenin, and comprises several signalling branches including Calcium dependant signalling, Planar Cell Polarity (PCP) molecules, small GTPases and C-Jun N-terminal kinases (JNK) signalling. These pathways are well described in numerous reviews such as (Clevers, 2006; Montcouquiol et al., 2006; Schlessinger et al., 2009).

In preferred embodiment, the Wnt signalling pathway is the canonical Wnt/beta catenin signalling pathway.

In another preferred embodiment, the Wnt signalling pathway is the Wnt/PCP signalling pathway.

In another preferred embodiment, the Wnt signalling pathway is the canonical Wnt/beta catenin signalling pathway and Wnt/PCP signalling pathway.

As used herein the term "activator" denotes a substance that enhances Wnt signalling activity. For example, for the canonical Wnt/beta-catenin signalling pathway, this activity can be measured by Wnt reporter activity using established multimers of LEF/TCF binding sites reporters, and/or inhibition of GSK-3ß, and/or activation of canonical Wnt target genes such as T, Tbx6, Msgn1, or Axin2.

As used herein the term "induced Paraxial Mesoderm progenitor cells" or "iPAM" refers to cells derived from any cell type but exhibiting characteristics of progenitor cells of the Paraxial Mesoderm. In one embodiment, the iPAM cells are characterized by the following properties:
a) they express biomarkers characteristic of Paraxial mesoderm progenitor cells such as Tbx6, EphrinA1, EphrinB2, EPHA4, PDGFRalpha, Sall1, Sall4, Dll1, Dll3, Papc (Pcdh8), Lfng, Hes7, Ripply1, Ripply2, Brachyury (T), Cdx2, Cdx4, Evx1, Cxcr4, Il17rd, Fgf8, Fgf17, Gbx2, Wnt3a, Wnt5b, Rspo3, SP5, SP8, Has2, Dkk1, Dact1, Pax3, Pax7, Mesp1, Mesp2 or Msgn1 genes. Preferentially Msgn1 gene as measured for example with a gene reporter assay comprising the Msgn1 promoter, and;
b) they are multipotent cells, capable of differentiating into at least skeletal, dermis or muscle cell lineages;
c) optionally, they may have long term self renewal properties, e.g., they can be maintained in culture more than 6 months.

The multipotency of said iPAM cells can be tested *in vitro,* e.g., by *in vitro* differentiation into skeletal, dermal or muscle cell lineages using the protocols described below, and in particular in the Examples.

As used herein, the term "multipotent" refers to cells that can differentiate in more than one cell lineage depending on the environmental and culture conditions. Contrary to embryonic stem cells which are pluripotent and can differentiate into all types of somatic cell lineages, the induced paraxial mesoderm progenitor cells of the present invention have limited differentiation capacity.

The term "pluripotent cells" as used herein refers to mammalian undifferentiated cells which can give rise to a variety of different cell lineages. Typically, pluripotent cells may express the following markers Oct4, SOX2, Nanog, SSEA 3 and 4, TRA 1/81, see International Stem Cell Initiative recommendations, 2007.

In one embodiment, the pluripotent cells are human pluripotent cells.

In another embodiment, the pluripotent cells are non-human mammalian pluripotent cells.

In one embodiment, the pluripotent cells are stem cells.

Typically, said stem cells are embryonic stem cells.

In a preferred embodiment, the pluripotent cells are human embryonic stem cells (hES cells). Typically, hES cell lines (Loser et al., 2010) such as the one described in the following table may be employed for the method of the invention:

| line | karyotype | passage available | country of origin | origin |
|---|---|---|---|---|
| **SA01** | 46XY | 25 | Sweden | Cellartis AB |
| **VUB01** | 46XY | 73 | Belgium | AZ-VUB Bruxel |
| **HUES 24,** | 46XY | 26 | USA | Harvard |
| **H1** | 46XY, 20q11.21 | 26 | USA | Wicell research Institute |
| **H9** | 46XX | 27 | USA | Wicell research Institute |
| **WT3** | 46XY | 35 | UK | UKSCB |
| **HUES1** | 46XX | 33 | USA | Harvard |

In one embodiment, the pluripotent cells are non-human embryonic stem cells, such as mouse stem cells, rodent stem cells or primate stem cells.

In one embodiment, the pluripotent cells are induced pluripotent stem cells (iPS). Induced pluripotent stem cells (iPS cells) are a type of pluripotent stem cells artificially derived from a non-pluripotent, typically an adult somatic cell, by inducing a "forced" expression of certain genes. iPS cells were first produced in 2006 from mouse cells (Takahashi and Yamanaka, 2006) and in 2007 from human cells (Takahashi et al., 2007; Yu et al., 2007).

In another embodiment, the activator of the canonical Wnt/beta catenin signalling pathway or the Wnt/PCP signalling pathway according to the invention is a member of the R-spondin family, originating from a vertebrate species or modified.

In another embodiment, the member of the R-spondin family is a member of the mammalian R-spondin family.

In a particular embodiment, the member of the R-spondin family according to the invention is selected in the group consisting of R-spondin 1, R-spondin 2, R-spondin 3 and R-spondin 4.

In a particular embodiment, the member of the R-spondin family according to the invention is R-spondin 3.

In a particular embodiment, the member of the R-spondin family according to the invention is R-spondin 2.

As used herein, the term "R-spondin3" or "R-spondin2" refers to members of the family of secreted proteins in vertebrates that activate Wnt signalling pathway.

An exemplary sequence for human R-spondin3 protein is deposited in the database under accession number NP_116173.2 (SEQ ID NO 1). An exemplary sequence for mouse R-spondin3 protein is deposited in the database under accession number NP_082627.3 (SEQ ID NO 2). An exemplary sequence for human R-spondin2 protein is deposited in the database under accession number NP_848660.3 (SEQ ID NO 3). An exemplary sequence for mouse R-spondin2 protein is deposited in the database under accession number NP_766403.1 (SEQ ID NO 4).

As used herein, the term "R-spondin3" also encompasses any functional variants of R-spondin3 wild type (naturally occurring) protein, provided that such functional variants retain the advantageous properties of differentiating factor for the purpose of the present invention. In one embodiment, said functional variants are functional homologues of R-spondin3 having at least 60%, 80%, 90% or at least 95% identity to the most closely related known natural R-spondin3 polypeptide sequence, for example, to human or mouse polypeptide R-spondin3 of SEQ ID NO:1 or SEQ ID NO:2 respectively, and retaining substantially the same Wnt activation activity as the related wild type protein. In another embodiment, said functional variants are fragments of R-spondin3, for example, comprising at least 50, 100, or 200 consecutive amino acids of a wild type R-spondin3 protein, and retaining substantially the same Wnt activation activity. In another embodiment, such functional variant can consist in R-spondin3 gene product isoforms such as the isoform 2 of the human R-spondin3 as described under the ref. Q9BXY4-2 and CAI20142.1 (SEQ ID NO 5).

As used herein, the term "R-spondin2" also encompasses any functional variants of R-spondin2 wild type (naturally occurring) protein, provided that such functional variants retain the advantageous properties of differentiating factor for the purpose of the present invention. In one embodiment, said functional variants are functional homologues of R-spondin2 having at least 60%, 80%, 90% or at least 95% identity to the most closely related known natural R-spondin2 polypeptide sequence, for example, to human or mouse polypeptide R-spondin2 of SEQ ID NO:3 or SEQ ID NO:4 respectively, and retaining substantially the same Wnt activation activity as the related wild type protein. In another embodiment, said functional variants are fragments of R-spondin2, for example, comprising at least 50, 100, or 200 consecutive amino acids of a wild type R-spondin2 protein, and retaining substantially the same Wnt activation activity. In another embodiment, said functional variants can consist in R-spondin2 gene product isoforms such as the isoform 2 or the isoform 3 of the human R-spondin2 such as described respectively under the ref. Q6UXX9-2 (SEQ ID NO 6) or under the ref. Q6UXX9-3 (SEQ ID NO 7).

As used herein, the percent identity between the two amino-acid sequences is a function of the number of identical positions shared by the sequences (i. e., % identity = # of identical positions/total # of positions x 100), taking into account the number of gaps, and the length of each gap, which need to be introduced for optimal alignment of the two sequences.

The comparison of sequences and determination of percent identity between two sequences can be accomplished using a mathematical algorithm, as described below.

The percent identity between two amino-acid sequences can be determined using the algorithm of E. Meyers and W. Miller (Comput. Appl. Biosci., 4:11-17, 1988) which has been incorporated into the ALIGN program (version 2.0), using a PAM120 weight residue table, a gap length penalty of 12 and a gap penalty of 4.

In another embodiment, the activator according to the invention is a combination of the R-spondin 3 and R-spondin 2.

In another embodiment, the activator according to the invention may be the human R-spondin-3 isoform 2 of sequence SEQ ID NO 5.

In another embodiment, the activator according to the invention may be the human R-spondin-2 isoform 2 of sequence SEQ ID NO 6, or the human R-spondin-2 isoform 3 of sequence SEQ ID NO 7.

In a preferred embodiment, the concentration of R-spondin3 used for culture of pluripotent cells is between 0.1 ng/ml and 500 ng/ml, preferably between 1 ng/ml and 500 ng/ml and more preferably between 5 ng/ml and 30 ng/ml.

In a preferred embodiment, the concentration of R-spondin2 used for culture of pluripotent cells is between 1 ng/ml and 500 ng/ml, preferably between 5 ng/ml and 30 ng/ml.

In a preferred embodiment, the concentration of R-spondin3 or R-spondin2 is 10 ng/ml. With a concentration of 10 ng/ml, more than 50% up to 70% of pluripotent cells are differentiated in iPAM

In another preferred embodiment, pluripotent cells are cultured with R-spondin3 or R-spondin2 during 1 to 15 days, or for a shorter time period. In a particular embodiment, pluripotent cells are cultured with R-spondin3 or/and R-spondin2 during at least 10 days at a concentration of 10 ng/ml.

In a preferred embodiment, the culture medium according to the invention may further comprise DMSO (Dimethyl sulfoxide) or an equivalent of the DMSO to improve the differentiation of pluripotent cells into iPAM

As used herein, the term "equivalent" means a substance exhibiting the same properties as DMSO.

In a preferred embodiment, the culture medium according to the invention comprises R-spondin 3 and DMSO to improve the differentiation of pluripotent cells into iPAM

In another preferred embodiment, the culture medium according to the invention comprises R-spondin 2 and DMSO to improve the differentiation of pluripotent cells into iPAM

In still another preferred embodiment, the culture medium according to the invention comprises R-spondin 3, R-spondin 2 and DMSO to improve the differentiation of pluripotent cells into iPAM

Vertebrate recombinant R-spondins can be purchased commercially, or produced as conditioned culture medium. This involves expressing a construct containing the coding sequence of a R-spondin protein into competent cells, such as COS cells. R-spondin protein is secreted in the culture medium. Conditioned medium can be applied directly to pluripotent cells or prediluted in basal medium.

In another preferred embodiment, the activator of the Wnt signalling pathway is an inhibitor of GSK3.

As used herein, the term "GSK3" for "Glycogen synthase kinase 3" denotes a serine/threonine protein kinase that mediates the addition of phosphate molecules on certain serine and threonine amino acids on particular cellular substrates. It is well known in the art that an inhibitor of GSK3 may activate the Wnt signalling pathway, see for example (Cohen and Goedert, 2004; Sato et al., 2004; Taelman et al., 2010; Wu and Pan, 2010).

In a preferred embodiment, the inhibitor of GSK3 is CHIR99021.

In another preferred embodiment, the following alternatives may be used for increasing the activity of R-spondin factor in the system:
1. enhancing endogenous expression of the gene encoding said R-spondin factor or a modified form of R-spondin,
2. allowing ectopic expression of said R-spondin factor by introducing an expression vector comprising a coding sequence of R-spondin factor operably linked to control sequences into the pluripotent cells to be differentiated, or by introducing in the cells coding RNA for R-spondin factor
3. introducing directly into the cells environment an appropriate amount of R-spondin factor, for example as recombinant R-spondin factor (family of R-spondin1, 2 ,3 and 4) in the culture medium, or conditioned medium, or as substrate coating.
4. activating or inhibiting endogeneous expression of a gene involved in R-spondin factor signalling in said target cells; or,
5. overexpressing proteins involved in controlling R-spondin factor expression level, maturation and overall regulation in said target cells.

In another preferred embodiment, introducing directly into the cells environment an appropriate amount of pharmacological GSK3 inhibitor, for example the chemical compound CHIR99021 is used as an alternative for increasing the activity of Wnt signalling pathway in the system, alone or in combination with R-spondin.

The invention relates to a composition for preparing iPAM cells from pluripotent cells wherein said composition comprises an effective amount of an activator of the Wnt signalling pathway according to the invention.

The invention also relates to a kit for preparing iPAM cells, said kit comprising:
a) an activator of the Wnt signaling pathway
b) optionally, instructions for preparing iPAM cells.

In a preferred embodiment, the activator is a member of the R-spondin family.

In another embodiment, the activator is selected from the group consisting of R-spondin 1, R-spondin 2, R-spondin 3 and R-spondin 4.

In another preferred embodiment, the activator is the R-spondin 2 or the R-spondin 3.

In another preferred embodiment, the activator is an inhibitor of GSK3 such as CHIR99021.

In a specific embodiment, said kit for preparing iPAM cells comprises,
a) a composition comprising members of the R-spondin family and
b) DMSO or an equivalent.

### Populations comprising iPAM cells obtainable from the methods of the invention

The invention further relates to populations comprising iPAM cells obtainable from the method as described above.

These populations typically may comprise other cell types in addition to iPAM cells. In one embodiment, the populations of the invention are characterized in that they comprise at least 10%, 20%, 30%, 40%, 50%, 60%, 70%, 80% and preferably at least 90% of cells that exhibit high expression of at least one biomarker characteristic of paraxial mesoderm progenitor cells, for example Msgn1 gene product.

Other biomarkers characteristic of paraxial mesoderm progenitor cells include, without limitation, one or more of the following proteins: Tbx6, EphrinA1, EphrinB2, EPHA4, PDGFRalpha, Sall1, Sall4, Dll1, Dll3, Papc (Pcdh8), Lfng, Hes7, Ripply1, Ripply2, Brachyury (T), Cdx2, Cdx4, Evx1, Cxcr4, Ill7rd, Fgf8, Fgf17, Gbx2, Wnt3a, Wnt5b, Rspo3, SP5, SP8, Has2, Dkk1, Dact1, Pax3, Pax7, Mesp1, Mesp2.

Any methods known in the art for measuring gene expression may be used, in particular, quantitative methods such as, real time quantitative PCR or microarrays, or methods using gene reporter expression, said gene reporter comprising Msgn1 promoter as described in the Examples, or qualitative methods such as immunostaining or cell sorting methods identifying cells exhibiting specific biomarkers, including cell surface markers.

As used herein, the Msgn1 gene refers to the gene encoding Mesogenin1. Examples of a nucleotide sequence of a gene encoding Mesogenin1 in mouse and human are given in SEQ ID NO:8 (NM_019544.1) and SEQ ID NO:9 (NM_001105569.1) respectively.

In one embodiment, expression of Msgn1 is considered high if expression is detectable in a quantitative assay for gene expression. In another embodiment, it is high if the expression level is significantly higher than the expression level observed in the original pluripotent cells, or in cells differentiating under non specific conditions such as Basal medium without LIF (Leukemia Inhibitory Factor) for mouse pluripotent cells or without FGF (Fibroblast Growth Factor) for human pluripotent cells. Expression levels between the control and the test cells may be normalized using constitutively expressed genes such as GAPDH or Beta Actin.

Populations comprising iPAM cells may be cultured indefinitely under appropriate growth conditions. Appropriate growth conditions may be established by the skilled person in the art based on established growth conditions for embryonic stem cells or induced pluripotent stem cells (iPS cells) for example or as described in the Examples below. Growth conditions may advantageously comprise for example the use of serum replacement medium, KSR (Gibco), ESGRO (Chemicon/Millipore) supplemented with growth factors like FGFs, WNTs, BMPs (Bone Morphogenetic Protein) or chemical compounds modulating the respective signalling pathways.

The iPAM cells may be purified or the populations may be enriched in iPAM cells by selecting cells expressing markers specific of iPAM cells. In one embodiment, markers specific of iPAM cells for purification or enrichment of a population of iPAM cells may be selected among one or more of the following markers: Msgn1, Tbx6, EphrinA1, EphrinB2, EPHA4, PDGFRalpha, Sall1, Sall4, Dll1, Dll3, Papc (Pcdh8), Lfng, Hes7, Ripply1, Ripply2, Brachyury (T), Cdx2, Cdx4, Evx1, Cxcr4, Il17rd, Fgf8, Fgf17, Gbx2, Wnt3a, Wnt5b, Rspo3, SP5, SP8, Has2, Dkk1, Dact1, Pax3, Pax7, Mesp1, Mesp2, or selected negatively with markers of other lineages/cell type such as neural fate.

Purification or iPAM enrichment may be achieved using cell sorting technologies, such as fluorescence activated cell sorting (FACS), or column affinity chromatography or magnetic beads comprising specific binders of said cell surface markers of iPAM cells, or fluorescent reporters for paraxial mesorderm progenitor makers. Another method consists in taking advantage of the differential adhesion properties of iPAM cells, by selective attachment on defined substrates.

After purification or enrichment, the population may thus comprise more than 10%, 20%, 30%, 40%, 50%, 60%; 70%, 80%, 90% or more than 95% of cells having a high expression of a biomarker characteristic of iPAM cells, for example, Msgn1 gene product.

In another preferred embodiment, the invention relates to a composition comprising a population of iPAM cells obtainable from the method as described above.

### Methods for preparing cell lineages by differentiation of iPAM cells

The iPAM cells may advantageously be cultured *in vitro* under differentiation conditions to generate skeletal muscle, bone, cartilage, dermal cells, as well as other derivatives of the paraxial mesoderm including but not restricted to adipocytes or endothelial cells.

Thus, the invention relates to a method for preparing populations comprising skeletal muscle, bone, cartilage, dermal cell, adipocytes or endothelial cells lineages said method comprising the steps of
(a) providing a population comprising iPAM cells; and,
(b) culturing said population comprising iPAM cells, under appropriate conditions for their differentiation into the desired cell lineages selected among the paraxial mesoderm derivatives which include skeletal muscle, bone, cartilage, dermal cell, adipocyte or endothelial cell lineages.

The invention further relates to a composition for preparing populations of cell lineages comprising iPAM cells according to the invention and appropriate conditions for their differentiation into the desired cell lineages.

In one specific embodiment, the present invention provides a method for preparing a population comprising skeletal muscle cell lineages, said method comprising the steps of
(a) providing a population comprising iPAM cells;
(b) culturing said population comprising iPAM cells in the presence of a differentiation medium comprising at least the following components:
   (i) an extracellular matrix material; and,
   (ii) compounds activating or inhibiting the signalling pathways known to control of the differentiation of said lineages which include but are not restricted to retinoic acid, BMP, TGFß (Transforming Growth Factorß), Hedgehog, Notch, FGF, Wnt, myostatin, insulin, PDGF, VEGF, MAPK, PI3K; and,
(c) optionally, culturing said population obtained from step (b) in a second differentiation medium comprising at least one or more compounds activating or inhibiting the Wnt, FGF, HGF (Hepatocyte growth factor), Activin, EGF (Epidermal growth factor), insulin, and IGF signalling pathways or compounds known to promote myogenic differentiation such as horse serum or transferrin,
   thereby obtaining a population comprising skeletal muscle cell lineages, that can be identified by markers such as Desmin, or Myosin Heavy Chain.

The use of engineered extracellular matrices or three dimensional scaffolds has been widely described in the Art (Metallo et al., 2007). In specific embodiments, the extracellular matrix material is selected from the group consisting of Collagen I, Collagen IV, Fibronectin, Laminin, gelatin, poly-lysine, PDMS and Matrigel.

The invention further relates to a composition for preparing skeletal muscle cell lineages from iPAM cells, characterized in that it further comprises:
i. an extracellular matrix material,
ii. at least one or more compounds activating or inhibiting the retinoic acid, BMP (Bone morphogenetic protein), TGFß, Hedgehog, Notch, FGF, Wnt, myostatin, insulin, PDGF (Platelet derived growth factor), VEGF (Vascular endothelial growth factor), MAPK, PI3K pathways. The composition further comprises at least another compound activating or inhibiting the Wnt, FGF, HGF, Activin, EGF, insulin, and IGF signalling pathways or compounds known to promote myogenic differentiation such as horse serum or transferrin.

In another embodiment, the present invention provides a method for preparing a population comprising dermal cell lineages, said method comprising the steps of culturing a population comprising iPAM cells in the presence of an efficient amount of at least one or more compounds activating or inhibiting BMP, TGFß, Wnt, FGF, EGF, retinoic acid, Notch and Hedgehog pathways. Dermal cells can be identified using markers such as Dermo-1.

The invention further relates to a composition for preparing dermal cell lineages from iPAM cells, characterized in that it further comprises at least one or more compounds activating or inhibiting BMP, TGFß, Wnt, FGF, EGF, retinoic acid, Notch and Hedgehog pathways.

In another specific embodiment, the present invention provides a method for preparing a population comprising bone or cartilage cell lineages, comprising the step of culturing a population comprising iPAM cells in the presence of an efficient amount of at least one or more compounds activating or inhibiting the retinoic acid, Wnt, Hedgehog, pTHRP, TGFß, BMP pathways, or compounds known to promote bone or cartilage differentiation such as dexamethasone, ascorbic acid, vitamin D3, and beta-glycerophosphate. Cartilage cells can be identified by classical staining such as Alcian Blue and bone cells with alizarin red or Von Kossa stain.

The invention further relates to a composition for preparing bone or cartilage cell lineages from iPAM cells, characterized in that it further comprises at least one or more compounds activating or inhibiting retinoic acid, Wnt, Hedgehog, pTHRP, TGFß, BMP pathways, or compounds known to promote bone or cartilage differentiation such as dexamethasone, ascorbic acid, vitamin D3 and beta-glycerophosphate.

In yet another embodiment, the present invention provides a method for preparing a population comprising adipocytes, said method comprising the steps of culturing the population comprising iPAM cells in the presence of an efficient amount of at least one or more compounds known to promote adipocyte differentiation including dexamethasone, isobutylxanthine and insulin. Adipocytes can be detected by OilRedO staining.

The invention further relates to a composition for preparing adipocytes from iPAM cells, characterized in that it further comprises at least one compound known to promote adipocyte differentiation including dexamethasone, isobutylxanthine and insulin.

In yet another embodiment, the present invention provides a method for preparing a population comprising endothelial cells, said method comprising the steps of culturing the population comprising iPAM cells in the presence of an efficient amount of at least one or more compounds activating or inhibiting the VEGF or FGF pathways. Endothelium can be detected by PECAM-1 (CD31) immunostaining.

The invention further relates to a composition for preparing endothelial cells from iPAM cells, characterized in that it further comprises at least one or more compounds activating or inhibiting the VEGF or FGF pathways.

Several examples of suitable conditions for differentiating iPAM cells into cartilage, muscles or endothelial cells are described in Examples below.

In another embodiment, the present invention provides populations comprising skeletal muscle, bone, cartilage, dermal cell, adipocytes or endothelial cells lineages as well as other derivatives derived from iPAM cells.

In a preferred embodiment, the present invention provides populations comprising skeletal muscle, bone, cartilage, dermal cell, adipocytes or endothelial cells lineages as well as other derivatives derived from iPAM cells obtained with a composition according to the invention.

In another embodiment, the invention relates to a composition comprising skeletal muscle, bone, cartilage, dermal cell, adipocyte or endothelial cell lineages obtainable by a method according to the invention.

### iPAM cells, population of cells derived from iPAM cells and uses thereof

Another aspect of the invention relates to the use of said populations comprising iPAM cells, or said populations comprising skeletal muscle, bone, cartilage or dermal cell lineages derived from differentiation of iPAM cells, but also adipose tissue and endothelial paraxial mesoderm derivatives, hereafter referred as the Populations of the Invention.

The Populations of the Invention may be used in a variety of applications, in particular, in research or therapeutic field.

One major field of application is cell therapy or regenerative medicine. For example, cells obtained from a patient suffering from a genetic defect may be cultured and genetically corrected according to methods known in the art, and subsequently reprogrammed into iPS cells and differentiated into iPAM or its derivatives for re-administration into the patient.

Similarly, regenerative medicine can be used to potentially cure any disease that results from malfunctioning, damaged or failing tissue by either regenerating the damaged tissues *in vivo* by direct *in vivo* implantation of a population comprising iPAM cells or their derivatives comprising appropriate progenitors or cell lineages.

Therefore, in one aspect, the invention relates to the iPAM cells or their derivatives or the Populations of the Invention for use as a cell therapy product for implanting into a mammal, for example human patient.

In one specific embodiment, the invention relates to a pharmaceutical composition comprising a population of iPAM cells obtained according to the invention. In another preferred embodiment, the invention relates to a pharmaceutical composition comprising a population of iPAM cells including for example at least 10², 10³, 10⁴, 10⁵, 10⁶, 10⁷, 10⁸, or at least 10⁹ Msgn1 expressing cells. In another embodiment, this composition comprises a pharmaceutically acceptable vehicle.

In one specific embodiment, the Populations of the Invention are used for the treatment of a muscle genetic disorder, for example Duchenne muscular dystrophy, or any other genetic muscular dystrophy.

In an embodiment, iPAM cells are co-cultured with various cell types to induce their differentiation toward the desired lineage. In another embodiment, iPAM cells are directly grafted into a recipient host. For regenerative medicine purposes, iPAM cells can be grafted after genetic correction by methods known in the art.

In another specific embodiment, the Populations of the Invention are used for the treatment of joint or cartilage or bone damages in orthopaedic surgery caused by aging, disease, or by physical stress such as occurs through injury or repetitive strain.

In another specific embodiment, the Populations of the Invention may also be used advantageously for the production of dermal tissues, for example, skin tissues, for use in regenerative medicine or in research, in particular in the cosmetic industry or for treatment of burns and plastic surgery.

In another preferred embodiment, the invention relates to a composition comprising the Populations of the Invention. The composition comprising the Population of the invention may be used in cell therapy or regenerative medicine.

The invention will be further illustrated by the following figures and examples. However, these examples and figures should not be interpreted in any way as limiting the scope of the present invention.

### FIGURES and TABLES:

**Table 1: Sequences of the invention**

| **Proteins** | **Bank Reference number** | **SEQ ID** | **Sequences** |
|---|---|---|---|
| hRspondin 3 | NP-116173.2 (CAI20141.1) or Q9BXY4-1 | SEQ ID NO1 | MHLRLISWLF IILNFMEYIG SQNASRGRRQ RRMHPNVSQG CQGGCATCSD YNGCLSCKPR LFFALERIGM KQIGVCLSSC PSGYYGTRYP DINKCTKCKA DCDTCFNKNF CTKCKSGFYL HLGKCLDNCP EGLEANNHTM ECVSIVHCEV SEWNPWSPCT KKGKTCGFKR GTETRVREII QHPSAKGNLC PPTNETRKCT VQRKKCQKGE RGKKGRERKR KKPNKGESKE AIPDSKSLES SKEIPEQREN KQQQKKRKVQ DKQKSVSVST VH |
| mRspondin 3 | NP-082627.3 | SEQ ID NO2 | MHLRLISCFF IILNFMEYIG SQNASRGRRQ RRMHPNVSQG CQGGCATCSD YNGCLSCKPR LFFVLERIGM KQIGVCLSSC PSGYYGTRYP DINKCTKCKV DCDTCFNKNF CTKCKSGFYL HLGKCLDSCP EGLEANNHTM ECVSIVHCEA SEWSPWSPCM KKGKTCGFKR GTETRVRDIL QHPSAKGNLC PPTSETRTCI VQRKKCSKGE RGKKGRERKR KKLNKEERKE TSSSSDSKGL ESSIETPDQQ ENKERQQQQK RRARDKQQKS VSVSTVH |
| hRspondin 2 | NP-848660.3 or Q6UXX9-1 | SEQ ID NO3 | MQFRLFSFAL IILNCMDYSH CQGNRWRRSK RASYVSNPIC KGCLSCSKDN GCSRCQQKLF FFLRREGMRQ YGECLHSCPS GYYGHRAPDM NRCARCRIEN CDSCFSKDFC TKCKVGFYLH RGRCFDECPD GFAPLEETME CVEGCEVGHW SEWGTCSRNN RTCGFKWGLE TRTRQIVKKP VKDTILCPTI AESRRCKMTM RHCPGGKRTP KAKEKRNKKK KRKLIERAQE QHSVFLATDR ANQ |
| mRspondin 2 | NP-766403.1 | SEQ ID NO4 | MRFCLFSFAL IILNCMDYSQ CQGNRWRRNK RASYVSNPIC KGCLSCSKDN GCSRCQQKLF FFLRREGMRQ YGECLHSCPS GYYGHRAPDM NRCARCRIEN CDSCFSKDFC TKCKVGFYLH RGRCFDECPD GFAPLDETME CVEGCEVGHW SEWGTCSRNN RTCGFKWGLE TRTRQIVKKP AKDTIPCPTI AESRRCKMAM RHCPGGKRTP KAKEKRNKKK RRKLIERAQE QHSVFLATDR VNQ |
| hRspondin 3 isoform2 | CA120142.1 or Q9BXY4-2 | SEQ ID NO5 | MHLRLISWLF IILNFMEYIG SQNASRGRRQ RRMHPNVSQG CQGGCATCSD YNGCLSCKPR LFFALERIGM KQIGVCLSSC PSGYYGTRYP DINKCTKCKA DCDTCFNKNF CTKCKSGFYL HLGKCLDNCP EGLEANNHTM ECVSIVHCEV SEWNPWSPCT KKGKTCGFKR GTETRVREII QHPSAKGNLC PPTNETRKCT VQRKKCQKGE RGKKGRERKR KKPNKGESKE AIPDSKSLES SKEIPEQREN KQQQKKRKVQ DKQKSGIEVT LAEGLTSVSQ RTQPTPCRRR YL |
| hRspondin 2 isoform2 | Q6UXX9.2 | SEQ ID NO6 | MRQYGECLHS CPSGYYGHRA PDMNRCARCR IENCDSCFSK DFCTKCKVGF YLHRGRCFDE CPDGFAPLEE TMECVEGCEV GHWSEWGTCS RNNRTCGFKW GLETRTRQIV KKPVKDTILC PTIAESRRCK MTMRHCPGGK RTPKAKEKRN KKKKRKLIER AQEQHSVFLA TDRANQ |
| hRspondin 2 isoform3 | Q6UXX9-3 | SEQ ID NO7 | FRLFSFAL IILNCMDYSH CQGNRWRRSK RGCRIENCDS CFSKDFCTKC KVGFYLHRGR CFDECPDGFA PLEETMECVG CEVGHWSEWG TCSRNNRTCG FKWGLETRTR QIVKKPVKDT ILCPTIAESR RCKMTMRHCP GGKRTPKAKE KRNKKKKRKL IERAQEQHSV FLATDRANQ |
| mMsgn1 | NM.019544.1 | SEQ ID NO8 | ATGGACAACC TGGGTGAGAC CTTCCTCAGC CTGGAGGATG GCCTGGACTC TTCTGACACC GCTGGTCTGC TGGCCTCCTG GGACTGGAAA AGCAGAGCCA GGCCCTTGGA GCTGGTCCAG GAGTCCCCCA CTCAAAGCCT CTCCCCAGCT CCTTCTCTGG AGTCCTACTC TGAGGTCGCA CTGCCCTGCG GGCACAGTGG GGCCAGCACA GGAGGCAGCG ATGGCTACGG CAGTCACGAG GCTGCCGGCT TAGTCGAGCT GGATTACAGC ATGTTGGCTT TTCAACCTCC CTATCTACAC ACTGCTGGTG GCCTCAAAGG CCAGAAAGGC AGCAAAGTCA AGATGTCTGT CCAGCGGAGA CGGAAGGCCA GCGAGAGAGA GAAACTCAGG ATGCGGACCT TAGCCGATGC CCTCCACACG CTCCGGAATT ACCTGCCGCC TGTCTACAGC CAGAGAGGCC AACCGCTCAC CAAGATCCAG ACACTCAAGT ACACCATCAA GTACATCGGG GAACTCACAG ACCTCCTCAA CAGCAGCGGG AGAGAGCCCA GGCCACAGAG TGTGTGA |
| hMsgn1 | NM00110556 9.1 | SEQ ID NO9 | ATGGACAACC TGCGCGAGAC TTTCCTCAGC CTCGAGGATG GCTTGGGCTC CTCTGACAGC CCTGGCCTGC TGTCTTCCTG GGACTGGAAG GACAGGGCAG GGCCCTTTGA GCTGAATCAG GCCTCCCCCT CTCAGAGCCT TTCCCCGGCT CCATCGCTGG AATCCTATTC TTCTTCTCCC TGTCCAGCTG TGGCTGGGCT GCCCTGTGAG CACGGCGGGG CCAGCAGTGG GGGCAGCGAA GGCTGCAGTG TCGGTGGGGC CAGTGGCCTG GTAGAGGTGG ACTACAATAT GTTAGCTTTC CAGCCCACCC ACCTTCAGGG CGGTGGTGGC CCCAAGGCCC AGAAGGGCAC CAAAGTCAGG ATGTCTGTCC AGCGGAGGCG GAAAGCCAGC GAGAGGGAGA AGCTCAGGAT GAGGACCTTG GCAGATGCCC TGCACACCCT CCGGAATTAC CTGCCACCTG TCTACAGCCA GAGAGGCCAG CCTCTCACCA AGATCCAGAC ACTCAAGTAC ACCATCAAGT ACATCGGGGA ACTCACAGAC CTCCTTAACC GCGGCAGAGA GCCCAGAGCC CAGAGCGCGT GA |

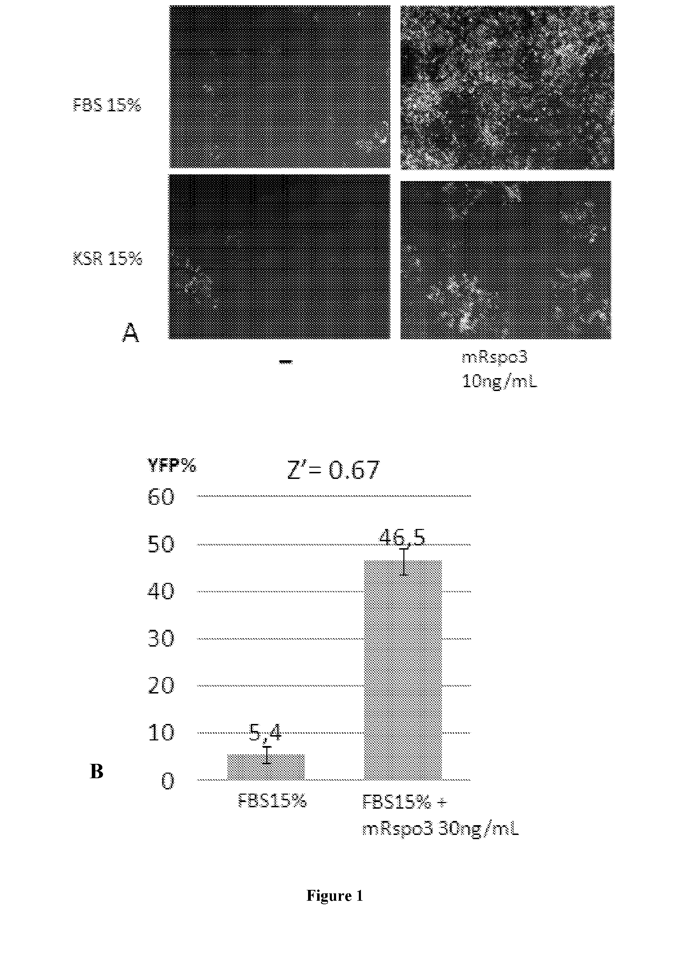

**Figure 1****: R-spondin induces iPAM fate.**
   (A) Comparison of fluorescent Msgn1 Reporter activation (YFP positive cells (YFP+ cells)) after 4 days of differentiation of mES cells (Msgn1RepV), under default culture conditions in 15% FBS or 15% KSR medium, with or without recombinant mouse Rspo3 (10ng/mL). YFP channel, 50X. (B) Robustness of iPAM cells induction in response to mouse Rspo3 in 15% FBS medium. Triplicate wells measurements by flow cytometry. Error bar is s.e.m.

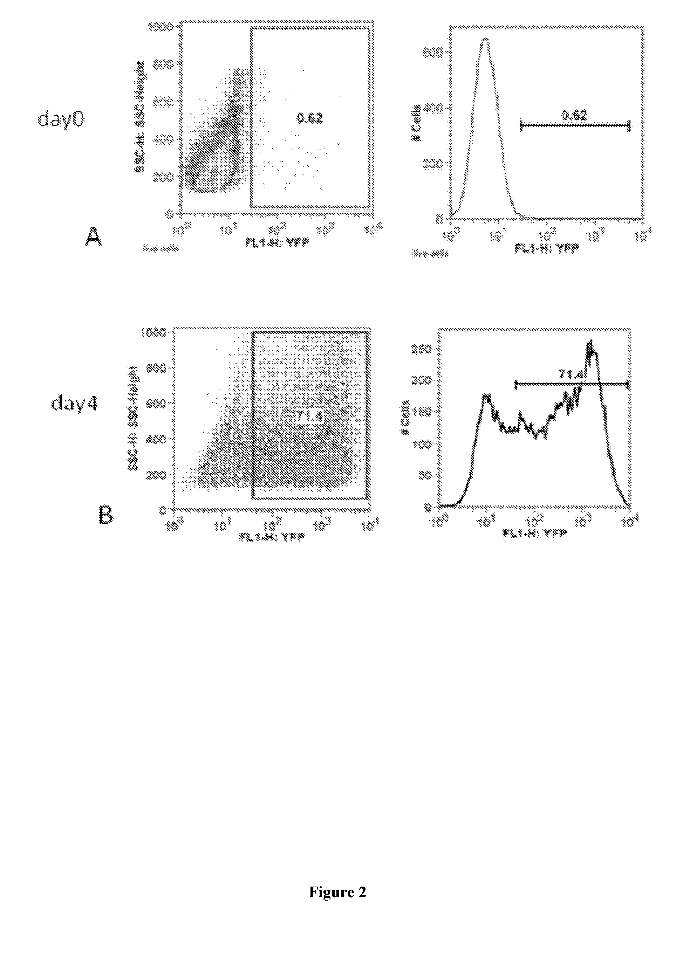

**Figure 2****: Flow-cytometry analysis of the induction of the Msgn1-YFP+ (iPAM) population upon treatment with Rspo3**
   (A) Flow-cytometry analysis on the Msgn1RepV mES cells at day0 of differentiation. YFP+ population represents less than 1%. (B) Flow-cytometry analysis at day 4 of differentiation in 15% FBS medium supplemented with R-spondin3 10ng/mL. YFP+ population represents more than 70% of the total population.

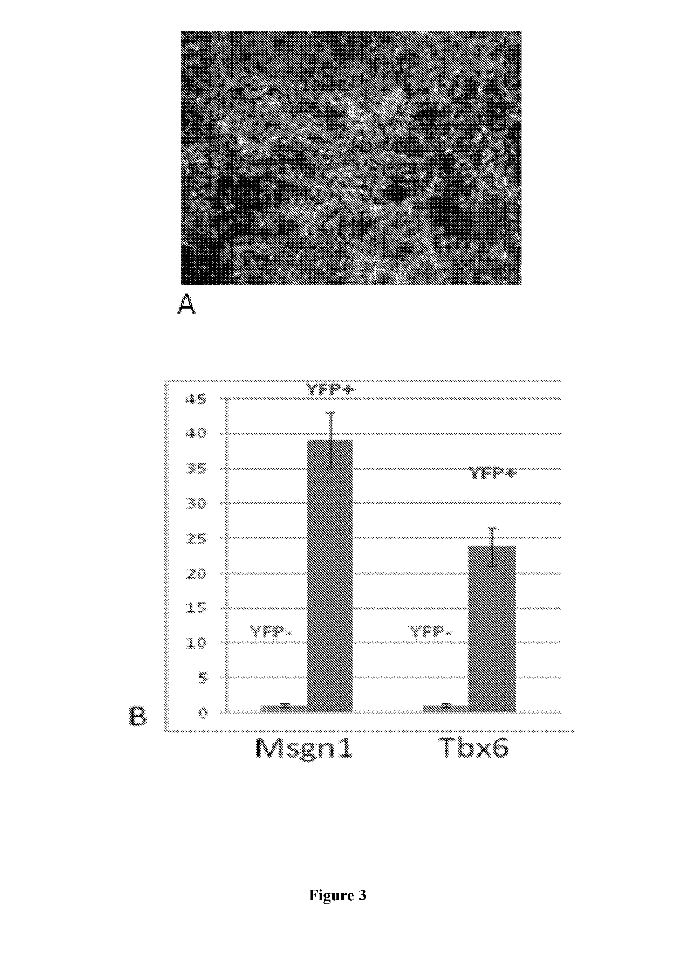

**Figure 3****: Paraxial mesoderm progenitors (iPAM) characterization**
   (A) Differentiation of mouse Msgn1RepV reporter mES cells into iPAM cells after 4 days in culture labeled with an anti-YFP antibody and co-stained with Hoechst, x10. (B) qRT - PCR analysis of FACS sorted iPAM YFP positive population for the paraxial mesoderm progenitors specific genes *Msgn1* and *Tbx6,* relative expression normalized to non iPAM YFP negative population expression level (fold enrichment).

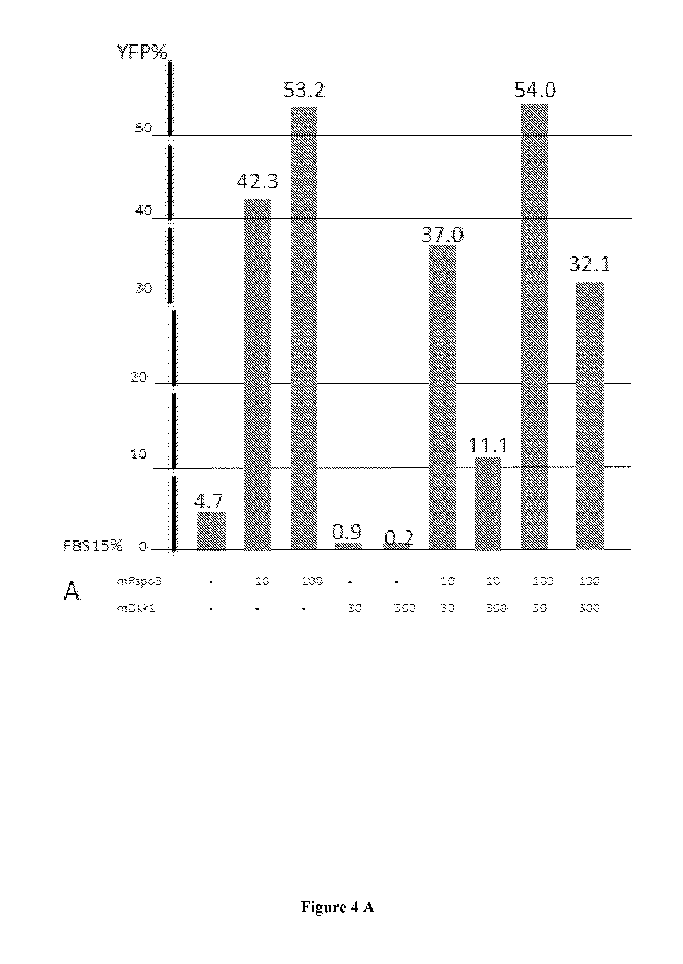

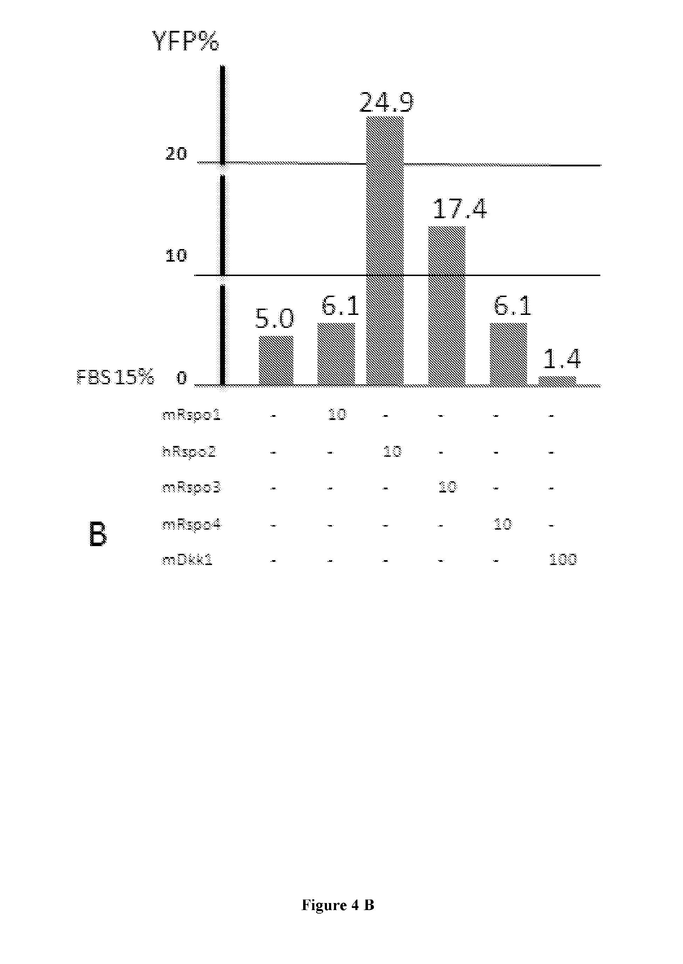

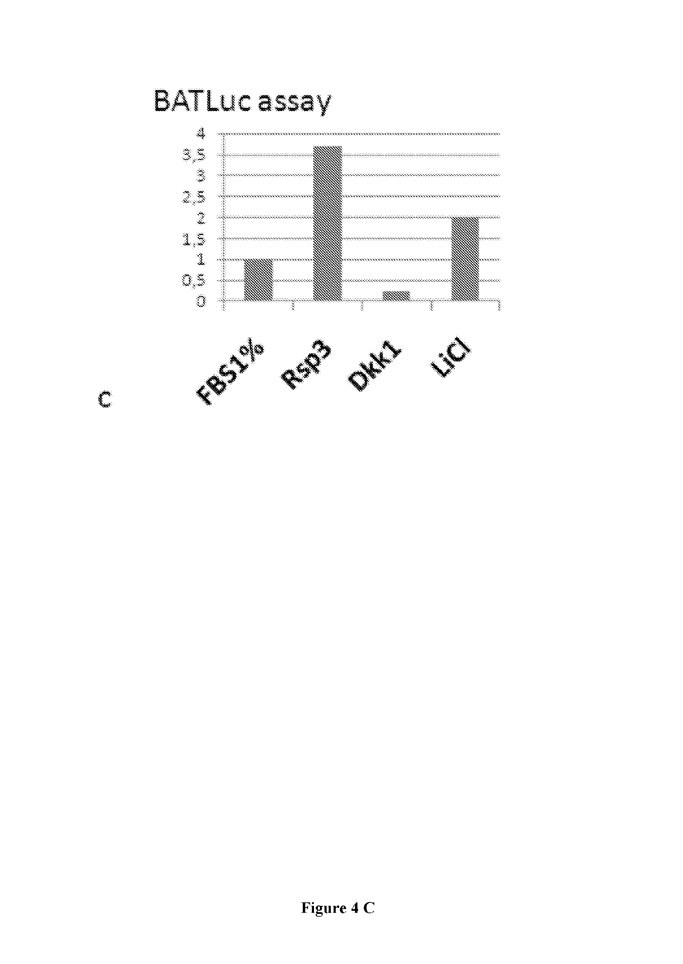

**Figure 4****: R-spondin activity in iPAM cells is mediated by canonical Wnt signaling.**
   (A) Comparison of the effect of different doses of Rspo3 on iPAM induction (%YFP positive cells) after 4 days of differentiation in 15% FBS medium in the presence or absence of the canonical Wnt inhibitor Dkk1. Concentrations in ng/mL. (B) Comparison of the efficiency of the four recombinant Rspo family members on iPAM induction (%YFP positive cells) after 4 days in differentiation 15% FBS medium. Concentrations in ng/mL. (C) Luciferase detection in Msgn1RepV reporter mES cells transfected with a Batluc reporter construct for canonical Wnt signaling activation and cultured in the presence of Rspo3 (10ng/mL), Dkk1 (50ng/mL) and LiCl (5mM) in low serum (1% FBS) containing medium. Treatment with Rspo3 strongly activates the canonical Wnt response in differentiating ES cells.
**Figure 5****: R-spondin activity can be mimicked by the GSK3beta inhibitor CHIR99021.** Comparison of the efficiency of Rspo3 and CHIR99021 on iPAM induction (%YFP positive cells) after 3 and 4 days in differentiation 15% FBS medium. Concentrations are in ng /mL for Rspo3 or in µM for CHIR99021.
**Figure 6****: DMSO has a positive effect on iPAM induction.** iPAM induction after 4 days of differentiation in 15% FBS medium containing 0.5% DMSO. Optimal iPAM induction is obtained by combining R-spondins and DMSO. Concentrations in ng/mL.
**Figure 7****: Rspo2 and 3 activity in defined medium**
   Analysis of the effect of recombinant mouse and human R-spondin 2 and 3 effect on iPAM induction (% of YFP positive cells) after 4 days of differentiation in 1% FBS (A) or 15% KSR (B) media. Concentrations in ng/mL.
**Figure 8****: Characterization of Populations of the Invention at day 18 of differentiation**
   From day 0 to day 4, mouse ES cells were differentiated in presence of Rspo3, followed by 15% FBS medium until day 18. Cell types were identified by tissue- specific antibody staining, namely Muscle (Desmin, green), Endothelium (PECAM1/CD31, green) and Cartilage (Alcian Blue).
**Figure 9****: Dermal and myogenic differentiation of the iPAM cells after 5 days of culture**
   ES cells were cultured for 4 days in FBS15%, DMSO 0.5% and 10 ng/ml Rspo3 and then switched to FBS 15% or FBS 1% or FBS1% plus Sonic Hedgehog (Shh) and Retinoic acid (F1ShhRA) or plus Shh, Noggin and LiCl (F1SNLi). Cells were harvested the next day and analyzed by qRT-PCR for the dermal marker Dermol (A) and the muscle marker Myf5 (B). Graphs show fold enrichment.

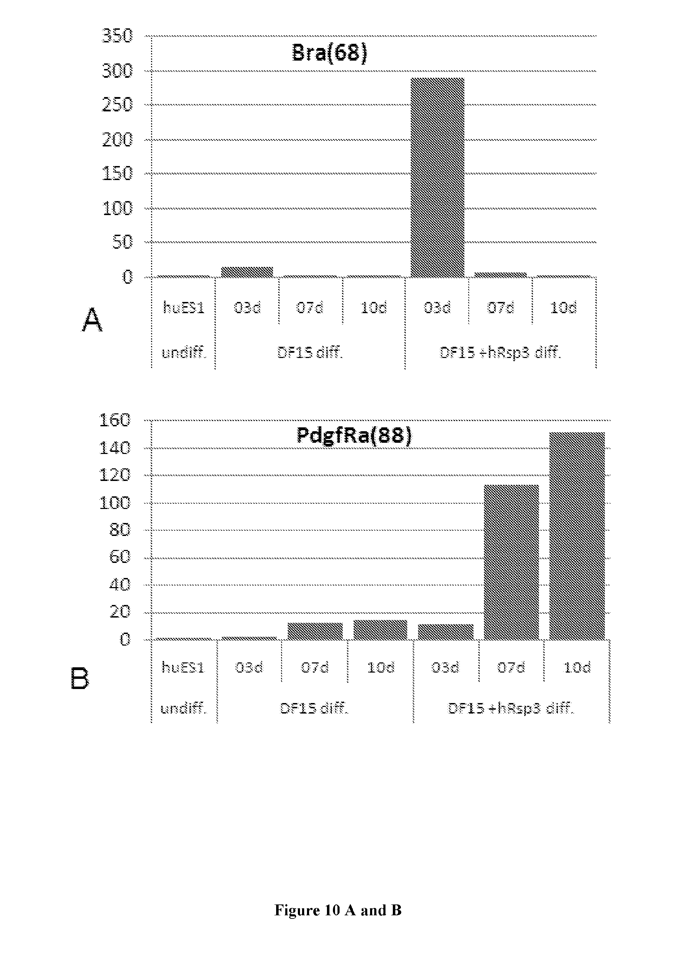

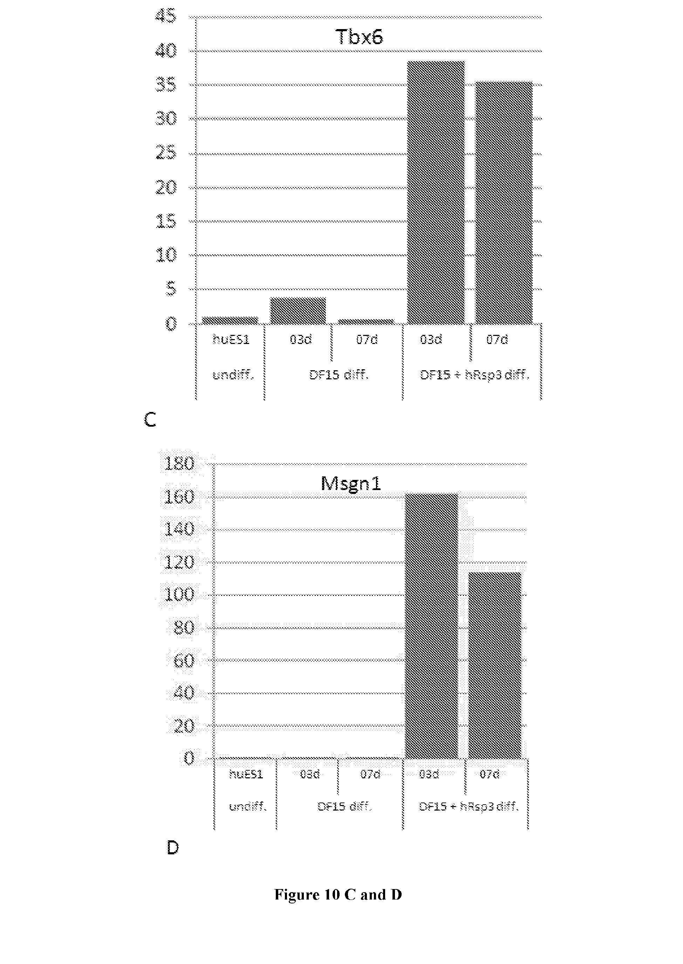

**Figure 10****: R-spondin induces iPAM fate in human ES cells**
   Comparison of the expression of paraxial mesoderm progenitor markers Brachyury (A), PDGFRa (B), Tbx6 (C), Msgn1 (D) measured by Q RT-PCR in HUES1 undifferentiated or cultured in 15% FBS containing medium with or without Rspo3 for up to 10 days. Relative expression to undifferentiated HUES1 cells is shown (fold induction)

### EXAMPLES:

### Material & Methods

### Cell culture

Undifferentiated mouse ES cells MsgnRepV (E14 derived) were maintained on gelatin-coated dishes in DMEM supplemented with 15% fetal bovine serum (FBS; from PAA), penicillin, streptomycin, 2mM L-Glutamine, 0.1mM non essential amino acids, 1mM sodium pyruvate, 0.2% β-mercaptoethanol and 1,500 U/mL LIF. ES cells were co-cultured with mytomicin- inactivated MEFs (feeders). Undifferentiated human ES cells were cultured on plates coated with matrigel (BD Biosciences) in mTeSR medium (STEMCELL Technologies). Cultures were maintained in an incubator in 5% CO2 at 37°C.

### Differentiation of ES cells

ES cells were trypsinized and plated at various densities in gelatin coated, feeder-free, 24 well plates directly in serum-based (15% FBS) or serum-replacement (15% KSR, Invitrogen) conditions supplemented with factors, and DMSO (Sigma). Recombinant proteins were obtained commercially (R&D) and stock solutions were prepared according to manufacturer's recommendation. The GSK-3ß inhibitor CHIR99021 was purchased from Stemgent and prepared according to the manufacturer's recommendations. Fluorescent reporter analysis and image acquisition were done on a Zeiss Axiovert system.

### FACS analysis and cell sorting

Cell cultures were dissociated by trypsinization, analyzed by flow cytometry on a FACScalibur (BD Biosciences) according to YFP expression. Data were further analyzed with MoFlo software (Beckman Coulter) and FlowJo software.

### Quantitative RT-PCR

Total RNA was extracted from ES cell cultures using a Trizol reagent (Invitrogen) or with the Rnaeasy plus mini-kit (Qiagen). RT-PCR was performed on 5ng total RNA using QuantiFast SYBR Green RT-PCR Kit (Qiagen), appropriate primers and run on a LightCycler 480II (Roche). GAPDH was used as the internal control.

### Differentiated Culture Phenotyping

Cell cultures were fixed with PFA 4% overnight at 4°C. Cells were incubated 20 minutes with a blocking solution composed of 1% fetal bovine serum and 0.1%Triton in Tris Buffered Saline (TBS). Primary antibodies incubation was performed overnight at 4°C and antibodies working dilutions were as follow: anti-GFP (Abcam) was 1:1,000 , anti- Desmin (DSHB) was 1 : 100, anti-CD31 (BD Pharmingen) was 1 : 100. After TBS washes, cells were incubated with AlexaFluor488-conjugated secondary antibodies (Molecular probes) at 1:500 for 30 minutes, and counterstained with Hoechst. Alcian Blue staining was done according to standard protocol

### Results

### Paraxial mesoderm progenitor (iPAM) cells in-vitro induction time window

We first aimed at identifying key molecular players promoting differentiation of the paraxial mesoderm lineage from ES cells. First, we investigated the time-course of paraxial mesoderm induction during mouse ES cell differentiation after formation of embryoid bodies, in DMEM based medium supplemented with 15% Fetal Bovine serum (FBS15%). Differentiation in paraxial mesoderm progenitor cells was characterized by activation of the *Brachyury*/*T, Tbx6,* and *Msgn1* markers detected by PCR. Our data suggest that between day 1 and 4 of culture, some differentiated cells are in a presomitic mesoderm -like stage.

### The Msgn1RepV reporter ES line characterization

In order to follow the differentiation of ES cells toward the first stage of paraxial mesoderm differentiation (ie presomitic fate), which represents the first step of skeletal muscle differentiation after acquisition of a mesodermal identity, we generated a transgenic mouse ES cell line harboring a fluorescent reporter specifically expressed in paraxial mesoderm progenitors. We used the promoter from the mouse *Msgn1,* a gene specific for the presomitic mesoderm, to drive the expression of Venus (a modified YFP). The transgenic Msgn1RepV (Mesogenin1 Reporter Venus) mouse ES cell line was subsequently validated using the tetraploid aggregation method to generate embryos entirely derived from the transgenic ES cells. As expected, transgenic mouse embryos exhibit fluorescently labeled paraxial mesoderm tissue, thus, validating the tissue specificity of Venus expression in the transgenic ES cell line.

### R-spondins identification

In order to optimize the differentiation conditions for paraxial mesoderm progenitors, we developed a manual screening assay, testing candidate growth factors and drugs interfering with various signaling pathways on ES cells. The Msgn1RepV reporter cells were plated at a defined density in 24-well plates coated with gelatin (0.1%). Two basal culture media were selected: a DMEM based medium containing 15% fetal bovine serum (FBS, high serum) and a defined serum-free medium containing 15% KSR (Invitrogen/Gibco). These basal media were supplemented with candidate factors on day 0 of differentiation. Control and experimental conditions were cultured in parallel. Cells were left to differentiate for three to four days with medium changed on day 2 or 3. Cell cultures were analyzed on day 3 and 4 of differentiation visually and by flow cytometry for YFP+ population quantification.

After 4 days of differentiation, control differentiation in 15% FBS results in a low and variable induction of YFP+ cells (typically 1 to 15% of the culture), and differentiation in defined medium 15% KSR (Invitrogen) results in an even lower induction (typically 1%). Among the set of candidates tested, we identified the secreted R-spondin3 protein as being able to increase dramatically the induction of YFP+ cells. In our assay, R-spondin3 at 10ng/mL is sufficient to increase significantly the induction of YFP+ cells both in FBS based medium and KSR based medium, up to 70% (Figure 1, 2 and 7). The R-spondin3 response saturated between 30 to 100ng/mL. While at day 0, the YFP+ population is <1% of the cells, in R-spondin3 supplemented medium differentiation, YFP+ population can represent more than 50%, up to 70% of the cells at day4 (Figure 2B). In human ES cells, induction of the paraxial mesoderm progenitor markers, Brachyury, PDGFRa, Tbx6 and Msgn1 is observed after 3 to 10 days of culture in 15% FBS containing medium when treating huES1 with R-spondin3 (Figure 10).

### Paraxial mesoderm progenitors characterization

To confirm that we obtained genuine paraxial mesoderm progenitor cells *in vitro* upon differentiation of ES cells, we sorted the YFP+ cell population after four days of differentiation in presence of R-spondin3 (Figure 3A) and analyzed the YFP+ versus YFP-cells by qRT-PCR for the key paraxial mesoderm markers *Msgn1* and *Tbx6* (Figure 3B). We confirmed that the YFP+ population strongly expresses the *Msgn1* endogenous gene, as well as *Tbx6,* demonstrating that we are able to generate paraxial mesoderm progenitors (i*PAM*) *in vitro.*

### R-spondin family and Wnt signaling

We next asked whether other members of the R-spondin family can induce iPAM (Msgn1-YFP+ paraxial mesoderm progenitors). ES cells were cultured in medium containing recombinant R-spondin proteins (R-spondins 1-4) supplemented with 15% FBS and allowed to differentiate for 4 days (Figure 4B). Two members of the family, R-spondin2 and R-spondin3, exhibit comparable activities and significantly increase the number of YFP+ cells. The activity of R-spondin family proteins has been associated with canonical Wnt/Beta catenin signaling (Kim et al., 2008; Nam et al., 2006) and more recently with Wnt/PCP signaling (Ohkawara et al., 2011). We analyzed the effect of the inhibition of canonical Wnt signaling using the secreted Dkk1 inhibitor, on R-spondin dependent differentiation (Figure 4A). Supplementation of the medium with the extracellular Wnt antagonist Dkk1 results in sharp decrease of YFP+ induction. Moreover, adding Dkk1 to FBS-containing medium blocks the effect of R-spondin3, suggesting that R-spondin3 effect is mediated by the Wnt canonical pathway. We also analyzed the expression of luciferase from a plasmid driven by a promoter responding to canonical Wnt signaling (BAT-luc) transfected in ES cells treated or not with R-spondin3, and with Dkk1 or with the compound LiCl which can activate the Wnt pathway (Figure 4C). Luciferase was strongly activated by R-spondin3 treatment suggesting that it activates the canonical Wnt pathway in this context.

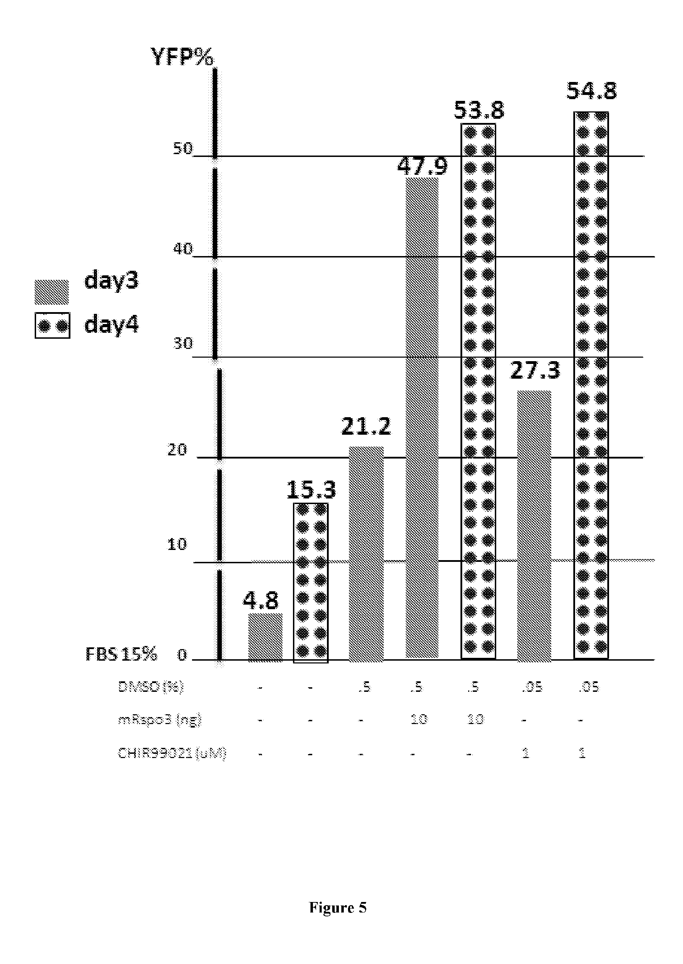

To further test whether R-spondin3 effect is mediated by the Wnt canonical pathway, we tested the effect of CHIR99021, a well described GSK-3ß inhibitor (Ring et al., 2003). Figure 5 shows that after 4 days, CHIR99021 is as efficient as Rspo-3 in inducing YFP+ cells, suggesting that R-spondin3 effect is mediated by activation of the canonical Wnt pathway.

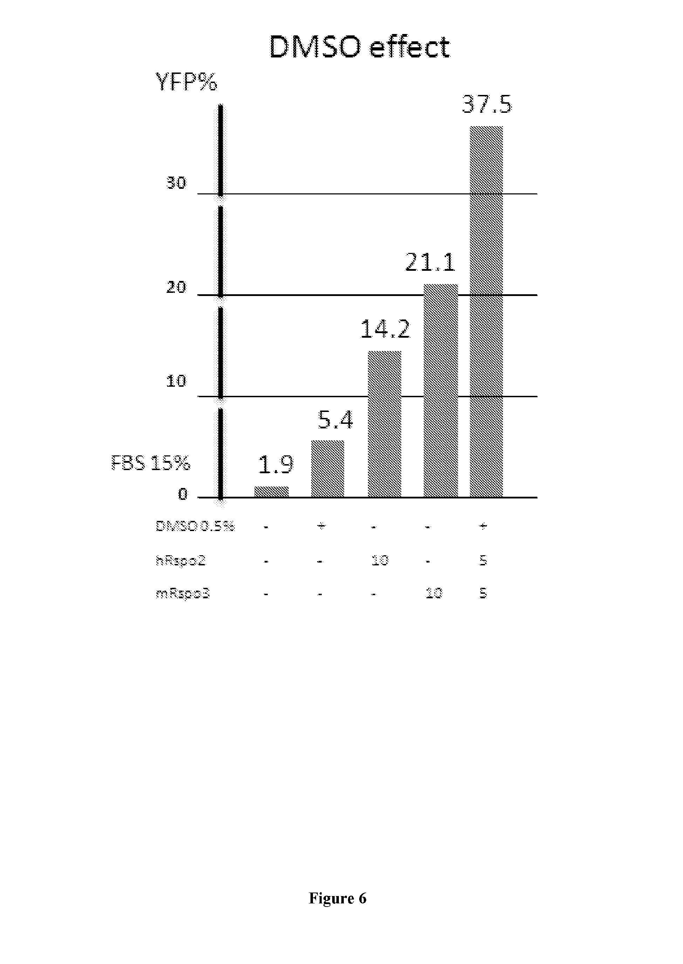

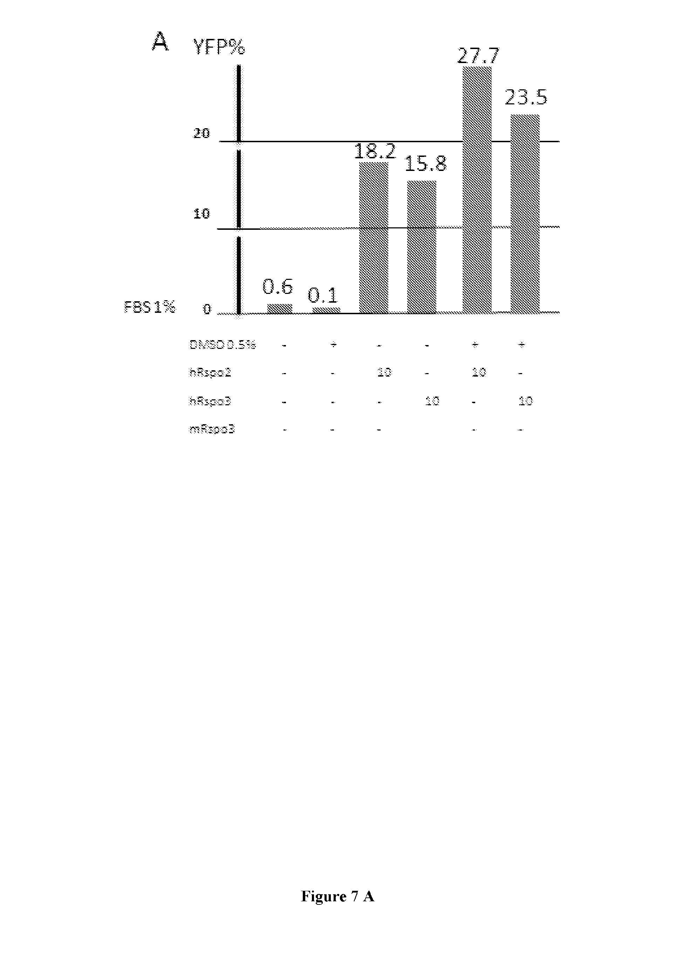

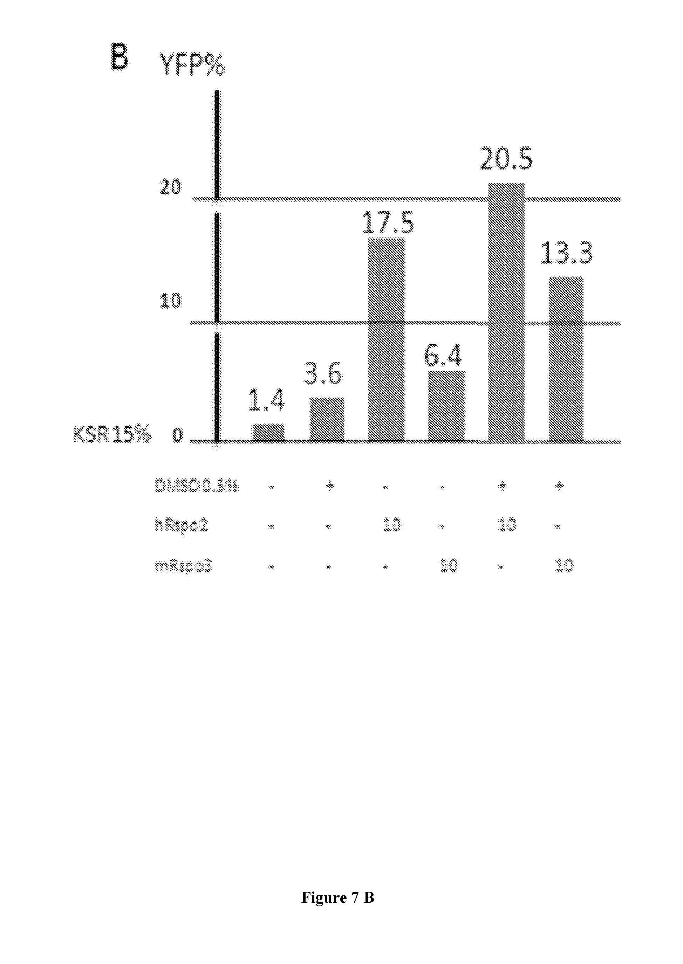

Dimethyl sulfoxide (DMSO) has been shown to promote differentiation of several cell types, notably mesoderm differentiation from the P19 Embryonic Carcinoma (EC) cell line (McBurney et al., 1982; Skerjanc, 1999). The exact mechanism of action of DMSO in cell culture is not known, and it has been hypothesized that DMSO modifies the plasma membrane properties, making the cells more responsive to extracellular signals present in the differentiation medium. Addition of 0.5% of DMSO to FBS-containing medium, results in an increase of YFP+ cells after 4 days in culture (Figure 5, 6 and 7), although this increase is modest compared to the increase due to the addition of R-spondin2 or R-spondin3, or both. Interestingly, the addition of R-spondins and DMSO synergizes to enhance paraxial mesoderm progenitors differentiation (Figure 5, 6 and7). Optimal conditions for paraxial mesoderm differentiation were observed when both DMSO and R-spondin 2 and/or 3 were combined (Figure 5, 6 and 7). Importantly, this effect is also seen in a serum-free, defined KSR based medium (Figure 7B).

### Paraxial mesoderm progenitors differentiation potential

We next explored the differentiation potential of the iPAM population of cells. *In vivo,* paraxial mesoderm progenitor cells are fated to become skeletal muscles, vertebral column tissues (cartilage, bone), dorsal dermis, endothelium, and other tissues such as adipose tissues.

Thus, we performed sequential differentiation protocols, aiming at first generating iPAM cells, and then differentiating them further in 15% FBS medium or by applying various described differentiation protocols (see below), in particular «Myogenic» and «Chondrogenic » media.

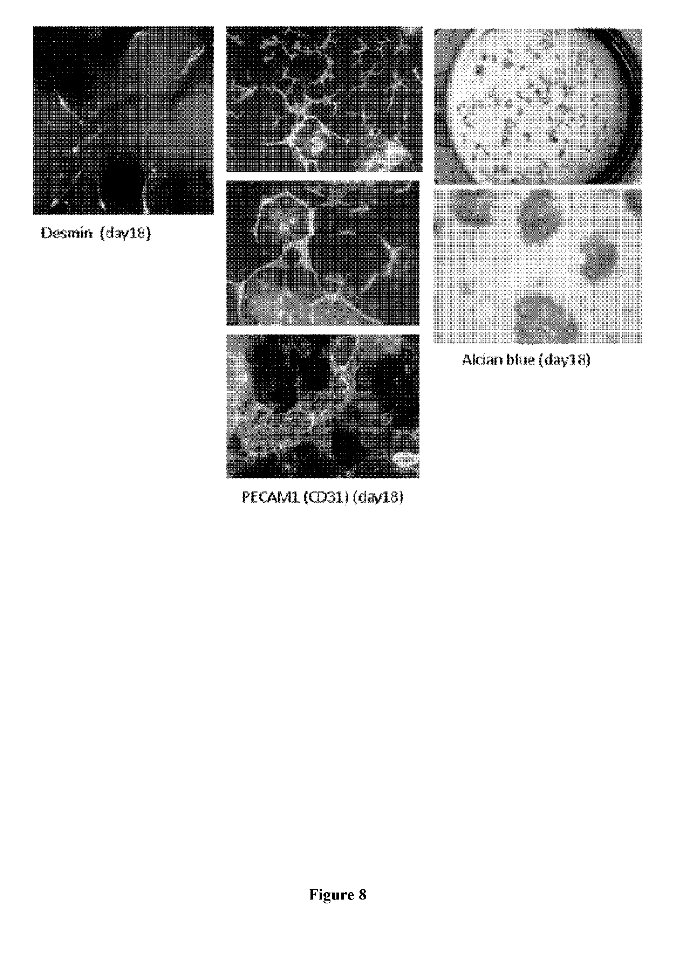

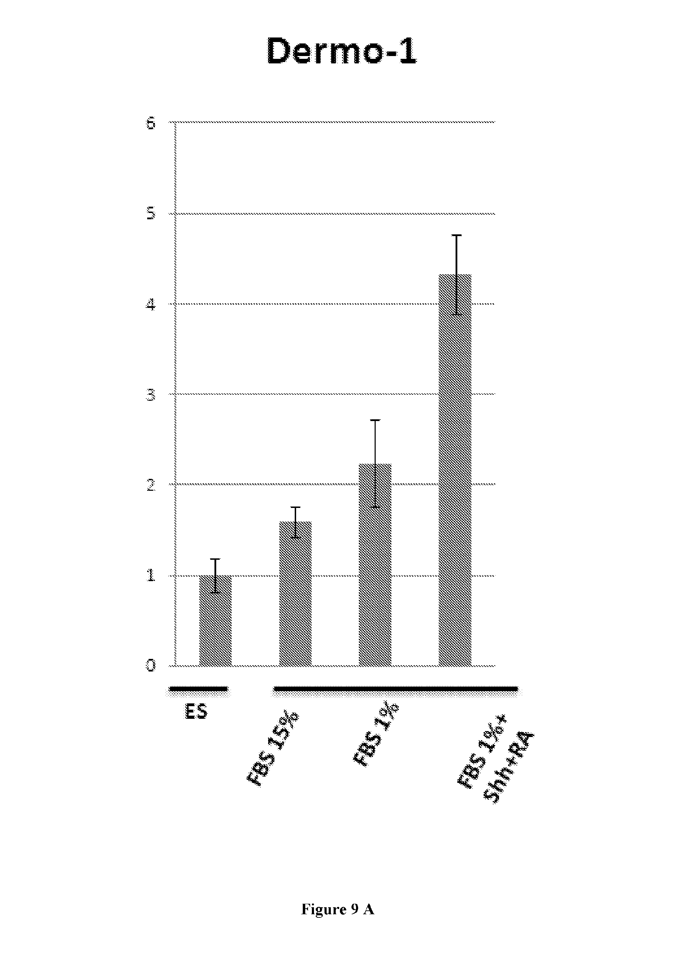

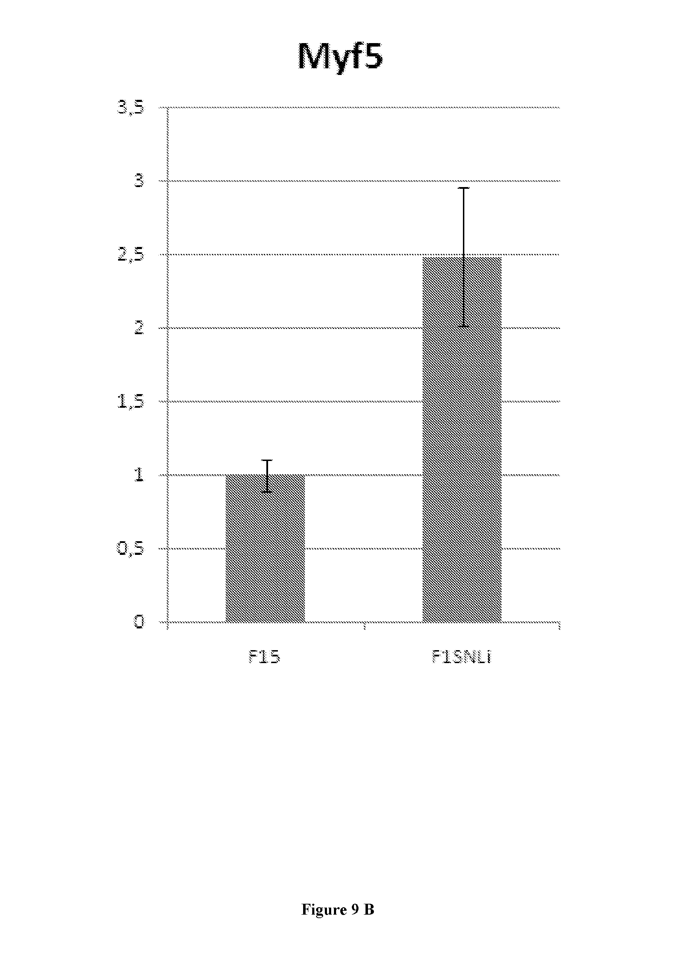

For example, between day 0 to day4, ES cells were exposed to optimized differentiating conditions (ie. R-spondin3 10ng/mL, DMSO 0.5%, in 15% FBS basal medium). On day 4, culture medium was changed and cells were exposed to particular differentiation media until day 18, with medium replacement every 3 days. At day 18, cell cultures were fixed and analyzed by tissue specific histochemical staining or immunofluorescence (Figure 8). Under optimized differentiation conditions, cell cultures were positive for Cartilage (Alcian blue positive nodules), Muscles (Desmin positive fibers) and Endothelium (CD31/PECAM1). Alternatively, after 4 days in differentiating conditions (ie. Rspo3 10ng/mL, DMSO 0.5%, in FBS15% basal medium), cells were switched to FBS 15% or FBS 1% or FBS1% plus Sonic Hedgehog (Shh) and Retinoic acid (F1ShhRA) or plus Shh, Noggin and LiCl (F1SNLi). Cells were harvested the next day and analyzed by qRT-PCR for the dermal marker Dermo1 and the muscle marker Myf5 (Figure 9). Significant activation of these markers was observed indicating differenciation of the iPAM cells toward the dermal and muscle lineages respectively.

### Myogenic protocol:

Alternatively, iPAM cells can be differentiated in two-dimensional culture into muscle cells using SFO3 medium complemented with BMP4, ActivinA and IGF-1 for 3 days, followed by 3 days of SFO3 medium complemented with LiCl and Shh.

iPAM cells can be cultured in a hanging drop for 3 days at 800cells/20uL in differentiation medium, composed of DMEM supplemented with 10% fetal calf serum (FCS), 5% horse serum (Sigma), 0.1 mM 2-mercaptoethanol, 0.1 mM nonessential aminoacids, and 50 ug/ml penicillin/streptomycin. After 3 days, the medium is changed and cell aggregates are transferred on a low attachement plate. At day 6, cells are plated and cultured in differentiation medium on plates coated with Matrigel (BD Bioscience, Bedford, MA, USA). Myogenic differentiation is achieved by withdrawal of FBS from confluent cells and addition of 10 ug/ml insulin, 5 ug/ml transferrin, and 2% horse serum.

iPAM cells can also be cultured for 3 weeks in Skeletal Muscle Cell Medium (Lonza, Chemicon) complemented with EGF, insulin, Fetuin, dexamethasone, and bFGF (100 ng/mL).

### Osteogenic protocol:

For skeletal lineages, iPAM cells are exposed to 200 ng/ml human or mouse recombinant BMP4 or a combination of 1 uM retinoic acid and 10 mM Lithium Chloride. Alternatively, cells are plated on gelatin-coated plates at a density of 1-3 × 10^3 per well (24-well plate) and cultured for 28 days in bone differentiation medium (DMEM, 10%FBS, 2 mM 1-Glutamine, 1 × Penicillin/streptomycin (P/S), 0.1 µM dexamethasone, 50 µM ascorbic acid 2-phosphate, 10 mM β-glycerophosphate, 10 ng/mL BMP4) in order to observe cells expressing bone specific markers or secreting alcian blue positive extracellular matrix. Differentiated skeletal cell lineages are identified using specific stainings for extracellular matrix components of bone and cartilage including alcian blue or alizarin red, as well as by immunofluorescence using chondrocyte- and/ or osteocyte specific antibodies.

iPAM cells can also be differentiated into the bone lineage using the following differentiation medium composed of DMEM, 10% FBS, 2 mM L-Glutamine, 1 × P/S, 0.1 mM Dexamethasone, 50 mM ascorbic acid 2-phosphate, 10 mM b-glycerophosphate, and 10 ng/mL BMP4, and vitamin D3 for 20 days, medium changed every 3 days. Bone formation can be confirmed by Alizarin red staining of the differentiating culture, well known in the art that results in the staining of differentiated bone in red color. Extracellular accumulation of calcium can also be visualized by von Kossa staining. Alternatively, differentiating cells can be lysed and assayed for ALP activity using BBTP reagent. Alternatively, differentiating cells can be analyzed for osteoblast lineage markers expression, for example Osterix(Osx) and Cbfa1/Runx2, alkaline phosphatase, collagen type I, osteocalcin, and osteopontin.

### Chondrogenic protocol:

For chondrogenic cell differentiation, iPAM cells are plated at a density of 8 x 10^4 per well (24-well plate) and cultured for 30 minutes in a 37C incubator in cartilage cell differentiation medium (αMEM, 10%FBS, 2 mM 1-Glutamine, 1 × P/S, 0.1 µM Dexamethasone, 170 µM ascorbic acid 2-phosphate). Next, an equal amount of cartilage cell differentiation medium with 10 ng/mL TGF beta3 is added to the well. After one week, the medium is replaced with cartilage differentiation medium supplemented with 10ng/mL Bmp2. After 21 days cartilaginous nodules secreting extracellular matrix can be observed. iPAM cells can also be differentiated into cartilage cells using a differentiation medium based on aMEM, 10%FBS, 2 mM L-Glutamine, 1 × P/S, 0.1 mM Dexamethasone, and 170 mM ascorbic acid 2-phosphate or DMEM supplemented with 0.1 mM dexamethasone, 0.17 mM ascorbic acid, 1.0 mM sodium pyruvate, 0.35mM L-proline, 1% insulin-transferrin sodium, 1.25 mg/ml bovine serum albumin, 5.33 ug/ml linoleic acid, and 0.01 ug/ml transforming growth factor-beta), as well as TGFb3 or BMP2. Cells are cultured for several weeks, with medium changed every 3 days. Differentiation can also be performed at high density on 3D scaffold such as Alginate beads in a DMEM based medium containing 10% FBS and antibiotic supplemented with 100 ng/ml recombinant human Bone Morphogenic Protein-2 (BMP-2) and 50 mg ascorbic acid. Cartilage formation can be confirmed by Alcian Blue staining of the differentiating culture, well known in the art that results in the staining of Muco-glycoproteins in blue color. Alternatively, a safranin O staining can be performed.

### Dermal fibroblast protocol:

iPAM cells can be differentiated into dermal fibroblasts by culturing them on a scaffold of collagen in medium containing a fibroblast growth factor such as bFGF (basic Fibroblast Growth Factor) or a member of the Wnt family of growth factors.

Next, to confirm that R-spondin also induces iPAM cells from human ES cells differentiation, HUES1 cells were plated has single cells and differentiated in 15% FBS containing medium with or without Rspo3. qRT-PCR time course analysis for paraxial mesoderm progenitor markers expression was performed (Figure 10). Strong activation of Msgn1 and Tbx6 during hES cells differentiation in presence of R-spondin3, demonstrate that iPAM cells can be differentiated from hES cells.

### REFERENCES:

Throughout this application, various references describe the state of the art to which this invention pertains. The disclosures of these references are hereby incorporated by reference into the present disclosure.
Barberi, T., Bradbury, M., Dincer, Z., Panagiotakos, G., Socci, N. D. and Studer, L. (2007). Derivation of engraftable skeletal myoblasts from human embryonic stem cells. Nat Med 13, 642-8.
Carmon, K. S., Gong, X., Lin, Q., Thomas, A. and Liu, Q. (2011). R-spondins function as ligands of the orphan receptors LGR4 and LGR5 to regulate Wnt/{beta}-catenin signaling. Proc Natl Acad Sci U S A 108, 11452-7.
Chal, J. and Pourquie, O. (2009). Patterning and Differentiation of the Vertebrate Spine. In The Skeletal System, (ed. O. Pourquie), pp. 41-116: Cold Spring Harbor Laboratory Press.
Chambers, I. (2004). The molecular basis of pluripotency in mouse embryonic stem cells. Cloning Stem Cells 6, 386-91.
Chapman, D. L., Agulnik, I., Hancock, S., Silver, L. M. and Papaioannou, V. E. (1996). Tbx6, a mouse T-Box gene implicated in paraxial mesoderm formation at gastrulation. Dev Biol 180, 534-42.
Clevers, H. (2006). Wnt/beta-catenin signaling in development and disease. Cell 127, 469-80.
Cohen, P. and Goedert, M. (2004). GSK3 inhibitors: development and therapeutic potential. Nat Rev Drug Discov 3, 479-87.
Darabi, R., Gehlbach, K., Bachoo, R. M., Kamath, S., Osawa, M., Kamm, K. E., Kyba, M. and Perlingeiro, R. C. (2008). Functional skeletal muscle regeneration from differentiating embryonic stem cells. Nat Med 14, 134-43.
Darabi, R., Santos, F. N., Filareto, A., Pan, W., Koene, R., Rudnicki, M. A., Kyba, M. and Perlingeiro, R. C. (2011). Assessment of the Myogenic Stem Cell Compartment Following Transplantation of Pax3/Pax7-Induced Embryonic Stem Cell-Derived Progenitors. Stem Cells.
de Lau, W., Barker, N., Low, T. Y., Koo, B. K., Li, V. S., Teunissen, H., Kujala, P., Haegebarth, A., Peters, P. J., van de Wetering, M. et al. (2011). Lgr5 homologues associate with Wnt receptors and mediate R-spondin signalling. Nature.
Dekel, I., Magal, Y., Pearson-White, S., Emerson, C. P. and Shani, M. (1992). Conditional conversion of ES cells to skeletal muscle by an exogenous MyoD1 gene. New Biol 4, 217-24.
Dimos, J. T., Rodolfa, K. T., Niakan, K. K., Weisenthal, L. M., Mitsumoto, H., Chung, W., Croft, G. F., Saphier, G., Leibel, R., Goland, R. et al. (2008). Induced pluripotent stem cells generated from patients with ALS can be differentiated into motor neurons. Science 321, 1218-21.
Dinsmore, J., Ratliff, J., Deacon, T., Pakzaban, P., Jacoby, D., Galpern, W. and Isacson, O. (1996). Embryonic stem cells differentiated in vitro as a novel source of cells for transplantation. Cell Transplant 5, 131-43.
Fukada, S., Higuchi, S., Segawa, M., Koda, K., Yamamoto, Y., Tsujikawa, K., Kohama, Y., Uezumi, A., Imamura, M., Miyagoe-Suzuki, Y. et al. (2004). Purification and cell-surface marker characterization of quiescent satellite cells from murine skeletal muscle by a novel monoclonal antibody. Exp Cell Res 296, 245-55.
Han, X. H., Jin, Y. R., Seto, M. and Yoon, J. K. (2011). A WNT/beta-catenin signaling activator, R-spondin, plays positive regulatory roles during skeletal myogenesis. J Biol Chem 286, 10649-59.
Hankenson, K. D., Sweetwyne, M. T., Shitaye, H. and Posey, K. L. (2010). Thrombospondins and novel TSR-containing proteins, R-spondins, regulate bone formation and remodeling. Curr Osteoporos Rep 8, 68-76.
Hirsinger, E., Jouve, C., Dubrulle, J. and Pourquie, O. (2000). Somite formation and patterning. Int Rev Cytol 198, 1-65.
Jin, Y. R., Turcotte, T. J., Crocker, A. L., Han, X. H. and Yoon, J. K. (2011). The canonical Wnt signaling activator, R-spondin2, regulates craniofacial patterning and morphogenesis within the branchial arch through ectodermal-mesenchymal interaction. Dev Biol 352, 1-13.
Kazanskaya, O., Glinka, A., del Barco Barrantes, I., Stannek, P., Niehrs, C. and Wu, W. (2004). R-Spondin2 is a secreted activator of Wnt/beta-catenin signaling and is required for Xenopus myogenesis. Dev Cell 7, 525-34.
Kazanskaya, O., Ohkawara, B., Heroult, M., Wu, W., Maltry, N., Augustin, H. G. and Niehrs, C. (2008). The Wnt signaling regulator R-spondin 3 promotes angioblast and vascular development. Development 135, 3655-64.
Kennedy, K. A., Porter, T., Mehta, V., Ryan, S. D., Price, F., Peshdary, V., Karamboulas, C., Savage, J., Drysdale, T. A., Li, S. C. et al. (2009). Retinoic acid enhances skeletal muscle progenitor formation and bypasses inhibition by bone morphogenetic protein 4 but not dominant negative beta-catenin. BMC Biol 7, 67.
Kim, K. A., Wagle, M., Tran, K., Zhan, X., Dixon, M. A., Liu, S., Gros, D., Korver, W., Yonkovich, S., Tomasevic, N. et al. (2008). R-Spondin family members regulate the Wnt pathway by a common mechanism. Mol Biol Cell 19, 2588-96.
Loser, P., Schirm, J., Guhr, A., Wobus, A. M. and Kurtz, A. (2010). Human embryonic stem cell lines and their use in international research. Stem Cells 28, 240-6.
McBurney, M. W., Jones-Villeneuve, E. M., Edwards, M. K. and Anderson, P. J. (1982). Control of muscle and neuronal differentiation in a cultured embryonal carcinoma cell line. Nature 299, 165-7.
Metallo, C. M., Mohr, J. C., Detzel, C. J., de Pablo, J. J., Van Wie, B. J. and Palecek, S. P. (2007). Engineering the stem cell microenvironment. Biotechnol Prog 23, 18-23.
Mizuno, Y., Chang, H., Umeda, K., Niwa, A., Iwasa, T., Awaya, T., Fukada, S., Yamamoto, H., Yamanaka, S., Nakahata, T. et al. (2010). Generation of skeletal muscle stem/progenitor cells from murine induced pluripotent stem cells. FASEB J 24, 2245-53.
Montcouquiol, M., Crenshaw, E. B., 3rd and Kelley, M. W. (2006). Noncanonical Wnt signaling and neural polarity. Annu Rev Neurosci 29, 363-86.
Nam, J. S., Turcotte, T. J., Smith, P. F., Choi, S. and Yoon, J. K. (2006). Mouse cristin/R-spondin family proteins are novel ligands for the Frizzled 8 and LRP6 receptors and activate beta-catenin-dependent gene expression. J Biol Chem 281, 13247-57.
Nam, J. S., Turcotte, T. J. and Yoon, J. K. (2007). Dynamic expression of R-spondin family genes in mouse development. Gene Expr Patterns 7, 306-12.
Ohkawara, B., Glinka, A. and Niehrs, C. (2011). Rspo3 binds syndecan 4 and induces Wnt/PCP signaling via clathrin-mediated endocytosis to promote morphogenesis. Dev Cell 20, 303-14.
Park, I. H., Arora, N., Huo, H., Maherali, N., Ahfeldt, T., Shimamura, A., Lensch, M. W., Cowan, C., Hochedlinger, K. and Daley, G. Q. (2008a). Disease-specific induced pluripotent stem cells. Cell 134, 877-86.
Park, I. H., Zhao, R., West, J. A., Yabuuchi, A., Huo, H., Ince, T. A., Lerou, P. H., Lensch, M. W. and Daley, G. Q. (2008b). Reprogramming of human somatic cells to pluripotency with defined factors. Nature 451, 141-6.
Prelle, K., Wobus, A. M., Krebs, O., Blum, W. F. and Wolf, E. (2000). Overexpression of insulin-like growth factor-II in mouse embryonic stem cells promotes myogenic differentiation. Biochem Biophys Res Commun 277, 631-8.
Ring, D. B., Johnson, K. W., Henriksen, E. J., Nuss, J. M., Goff, D., Kinnick, T. R., Ma, S. T., Reeder, J. W., Samuels, I., Slabiak, T. et al. (2003). Selective glycogen synthase kinase 3 inhibitors potentiate insulin activation of glucose transport and utilization in vitro and in vivo. Diabetes 52, 588-95.
Rohwedel, J., Maltsev, V., Bober, E., Arnold, H. H., Hescheler, J. and Wobus, A. M. (1994). Muscle cell differentiation of embryonic stem cells reflects myogenesis in vivo: developmentally regulated expression of myogenic determination genes and functional expression of ionic currents. Dev Biol 164, 87-101.
Sakurai, H., Inami, Y., Tamamura, Y., Yoshikai, T., Sehara-Fujisawa, A. and Isobe, K. (2009). Bidirectional induction toward paraxial mesodermal derivatives from mouse ES cells in chemically defined medium. Stem Cell Res 3, 157-69.
Sakurai, H., Okawa, Y., Inami, Y., Nishio, N. and Isobe, K. (2008). Paraxial mesodermal progenitors derived from mouse embryonic stem cells contribute to muscle regeneration via differentiation into muscle satellite cells. Stem Cells 26, 1865-73.
Sato, N., Meijer, L., Skaltsounis, L., Greengard, P. and Brivanlou, A. H. (2004). Maintenance of pluripotency in human and mouse embryonic stem cells through activation of Wnt signaling by a pharmacological GSK-3-specific inhibitor. Nat Med 10, 55-63.
Schlessinger, K., Hall, A. and Tolwinski, N. (2009). Wnt signaling pathways meet Rho GTPases. Genes Dev 23, 265-77.
Shani, M., Faerman, A., Emerson, C. P., Pearson-White, S., Dekel, I. and Magal, Y. (1992). The consequences of a constitutive expression of MyoD1 in ES cells and mouse embryos. Symp Soc Exp Biol 46, 19-36.
Skerjanc, I. S. (1999). Cardiac and skeletal muscle development in P19 embryonal carcinoma cells. Trends Cardiovasc Med 9, 139-43.
Taelman, V. F., Dobrowolski, R., Plouhinec, J. L., Fuentealba, L. C., Vorwald, P. P., Gumper, I., Sabatini, D. D. and De Robertis, E. M. (2010). Wnt signaling requires sequestration of glycogen synthase kinase 3 inside multivesicular endosomes. Cell 143, 1136-48.
Takahashi, K., Tanabe, K., Ohnuki, M., Narita, M., Ichisaka, T., Tomoda, K. and Yamanaka, S. (2007). Induction of pluripotent stem cells from adult human fibroblasts by defined factors. Cell 131, 861-72.
Takahashi, K. and Yamanaka, S. (2006). Induction of pluripotent stem cells from mouse embryonic and adult fibroblast cultures by defined factors. Cell 126, 663-76.
Wittler, L., Shin, E. H., Grote, P., Kispert, A., Beckers, A., Gossler, A., Werber, M. and Herrmann, B. G. (2007). Expression of Msgn1 in the presomitic mesoderm is controlled by synergism of WNT signalling and Tbx6. EMBO Rep 8, 784-9.
Wu, D. and Pan, W. (2010). GSK3: a multifaceted kinase in Wnt signaling. Trends Biochem Sci 35, 161-8.
Yoon, J. K. and Wold, B. (2000). The bHLH regulator pMesogenin1 is required for maturation and segmentation of paraxial mesoderm. Genes Dev 14, 3204-14.
Yu, J., Vodyanik, M. A., Smuga-Otto, K., Antosiewicz-Bourget, J., Frane, J. L., Tian, S., Nie, J., Jonsdottir, G. A., Ruotti, V., Stewart, R. et al. (2007). Induced pluripotent stem cell lines derived from human somatic cells. Science 318, 1917-20.

## Claims

1. An *ex vivo* method for preparing a population of induced paraxial mesoderm progenitor (iPAM) cells, said method comprising the step of culturing pluripotent cells in an appropriate culture medium comprising an effective amount of an activator of the Wnt signalling pathway.

2. An *ex vivo* method according to claim 1, wherein said Wnt signalling pathway is the canonical Wnt/beta catenin signalling pathway and/or the Wnt/PCP signalling pathway.

3. An *ex vivo* method according to one of the claims 1 to 2, wherein the activator of said Wnt signalling pathway is a member of the R-spondin family.

4. An *ex vivo* method according to the claim 3 wherein the said member of the R-spondin family is selected in the group consisting of R-spondin 3, R-spondin2, or a combination of said R-spondin 3 and R-spondin 2.

5. An *ex vivo* method according to claim 4, **characterized in that** said R-spondin-3 is the human R-spondin-3 of sequence SEQ ID NO 1 or the human R-spondin-3 isoform 2 of sequence SEQ ID NO 5.

6. An *ex vivo* method according to claim 4, **characterized in that** said R-spondin-2 is the human R-spondin-2 of sequence SEQ ID No 3, the human R-spondin-2 isoform 2 of sequence SEQ ID NO 6, or the human R-spondin-2 isoform 3 of sequence SEQ ID NO 7.

7. An *ex vivo* method according to one of the claims 1 to 2, wherein the activator of said Wnt signalling pathway is an inhibitor of GSK3.

8. An *ex vivo* method according to one of claim 1 to 7 wherein said appropriate culture medium further comprises DMSO, or an equivalent.

9. The method according to one of the claims 1 to 8 wherein the pluripotent stem cells are mouse or human embryonic stem cells or iPS cells

10. A population comprising iPAM cells obtainable from the method according to claims 1 to 9.

11. A population according to claim 10 **characterized in that** at least 10%, 20%, 30%, 40%, 50%, 60%, 70%, 80% and preferably 90% of the cells in said population, exhibit a high expression of biomarker characteristic of paraxial mesoderm progenitor cells, for example, Msgn1 gene product.

12. A method for preparing populations comprising skeletal muscle, bone, cartilage, dermal cell, adipocytes or endothelial cells lineages, said method comprising the steps of
(a) providing a population comprising iPAM cells; and,
(b) culturing said population comprising iPAM cells, under appropriate conditions for their differentiation into the desired cell lineages selected among the paraxial mesoderm derivatives which include skeletal muscle, bone, cartilage, dermal cell, adipocytes or endothelial cells lineages.

13. The method according to claim 12, for preparing populations comprising skeletal muscle cell lineages, said method comprising the steps of
(a) providing a population comprising iPAM cells;
(b) culturing said population comprising iPAM cells in the presence of a differentiation medium comprising at least the following components:
i. an extracellular matrix material; and,
ii. compounds activating or inhibiting the signalling pathways known to control of the differentiation of said lineages which include but are not restricted to retinoic acid, BMP, TGFß (Transforming Growth Factorß), Hedgehog, Notch, FGF, Wnt, myostatin, insulin, PDGF, VEGF, MAPK, PI3K; and,
(c) optionally, culturing said population obtained from step (b) in a second differentiation medium comprising at least one or more compounds activating or inhibiting the Wnt, FGF, HGF (Hepatocyte growth factor), Activin, EGF (Epidermal growth factor), insulin, and IGF signalling pathways or compounds known to promote myogenic differentiation such as horse serum or transferrin,
thereby obtaining a population comprising skeletal muscle cell lineages, that can be identified by markers such as Desmin, or Myosin Heavy Chain.

14. The method according to claim 12 for preparing a population comprising dermal cell lineages, said method comprising the steps of culturing a population comprising iPAM cells in the presence of an efficient amount of at least one or more compounds activating or inhibiting the BMP, TGFß, Wnt, FGF, EGF, retinoic acid, Notch and Hedgehog pathways. Dermal cells can be identified using markers such as Dermo-1.

15. The method according to claim 12 for preparing a population comprising bone or cartilage cell lineages, comprising the step of culturing a population comprising iPAM cells in the presence of an efficient amount of at least one or more compounds activating or inhibiting retinoic acid, Wnt, Hedgehog, pTHRP, TGFß, BMP pathways, or compounds known to promote bone or cartilage differentiation such as dexamethasone, ascorbic acid, vitamin D3, and beta-glycerophosphate. Cartilage cells can be identified by classical staining such as Alcian Blue and bone cells with alizarin red or Von Kossa stain.
